(19) Europäisches Patentamt / European Patent Office / Office européen des brevets

(11) **EP 2 134 732 B1**

(12) **EUROPÄISCHE PATENTSCHRIFT**

(45) Veröffentlichungstag und Bekanntmachung des Hinweises auf die Patenterteilung:
**24.11.2010 Patentblatt 2010/47**

(21) Anmeldenummer: **07818668.1**

(22) Anmeldetag: **04.10.2007**

(51) Int Cl.:
***C07F 9/655*** *(2006.01)*

(86) Internationale Anmeldenummer:
**PCT/EP2007/008589**

(87) Internationale Veröffentlichungsnummer:
**WO 2008/052631 (08.05.2008 Gazette 2008/19)**

(54) **[(4-OXO-4H-CHROMEN-3-YL)-HYDROXYMETHYL]- ODER [(4-OXO-4H-CHROMEN-3-YL)-METHYL]-PHOSPHONSÄUREDERIVATE**

[(4-OXO-4H-CHROMEN-3-YL)-HYDROXY METHYL]- OR [(4-OXO-4H-CHROMEN-3-YL)-METHYL]-PHOSPHONIC ACID DERIVATES

DÉRIVÉS D'ACIDE [(4-OXO-4H-CHROMÉNO-3-YL)-HYDROXYMÉTHYL]-PHOSPHONIQUE OU [(4-OXO-4H-CHROMÉNO-3-YL)-MÉTHYL]-PHOSPHONIQUE

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **28.10.2006 DE 102006050925**

(43) Veröffentlichungstag der Anmeldung:
**23.12.2009 Patentblatt 2009/52**

(73) Patentinhaber: **Merck Patent GmbH**
**64293 Darmstadt (DE)**

(72) Erfinder:
- **CAROLA, Christophe**
  **69120 Heidelberg (DE)**
- **BUCHHOLZ, Herwig**
  **60599 Frankfurt (DE)**

(56) Entgegenhaltungen:
**JP-A- 10 237 081 JP-A- 55 143 908**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 134 732 B1

Printed by Jouve, 75001 PARIS (FR)

## Beschreibung

Die vorliegende Erfindung betrifft [(4-Oxo-4H-chromen-3-yl)-hydroxymethyl]- oder [(4-Oxo-4H-chromen-3-yl)-methyl-phosphonsäure, deren Derivate und/oder deren Salze, Zubereitungen und Gemische enthaltend diese Verbindungen, sowie deren Herstellung und Verwendung.

Ein Einsatzgebiet der erfindungsgemäßen Verbindungen ist beispielsweise die Kosmetik. Aufgabe pflegender Kosmetik ist es, nach Möglichkeit den Eindruck einer jugendlichen Haut zu erhalten. Prinzipiell stehen verschiedene Wege offen, um diesen Weg zu erreichen. So können bereits vorhandene Schädigungen der Haut, wie unregelmäßige Pigmentierung oder Faltenbildung, durch abdeckende Puder oder Cremes ausgeglichen werden. Ein anderer Ansatzpunkt ist, die Haut vor Umwelteinflüssen zu schützen, die zu einer dauerhaften Schädigung und damit Alterung der Haut führen. Die Idee ist also, vorbeugend einzugreifen und dadurch den Alterungsprozess hinauszuzögern. Ein Beispiel sind hierfür die bereits erwähnten UV-Filter, welche durch Absorption bestimmter Wellenlängenbereiche eine Schädigung der Haut vermeiden oder zumindest vermindern. Während bei UV-Filtern das schädigende Ereignis, die UV-Strahlung, von der Haut abgeschirmt wird, versucht man bei einem weiteren Weg, die natürlichen Abwehr- bzw. Reparaturmechanismen der Haut gegen das schädigende Ereignis zu unterstützen. Schließlich verfolgt man als weiteren Ansatzpunkt die mit zunehmendem Alter sich abschwächenden Abwehrfunktionen der Haut gegen schädigende Einflüsse auszugleichen, indem Substanzen von außen zugeführt werden, die diese nachlassende Abwehr- bzw. Reparaturfunktion ersetzen können. Beispielsweise besitzt die Haut die Fähigkeit, Radikale, die durch äußere oder innere Stressfaktoren erzeugt werden, abzufangen. Diese Fähigkeit schwächt sich mit zunehmendem Alter ab, wodurch sich der Alterungsprozess mit zunehmendem Alter beschleunigt.

Eine mehr oder minder stark ausgeprägte Sonnenbräune der Haut gilt in der modernen Gesellschaft als attraktiv und als Ausdruck von Dynamik und Sportlichkeit. Neben dieser erwünschten Wirkung der Sonne auf die Haut treten eine Reihe von unerwünschten Nebenwirkungen auf, wie Sonnenbrand oder vorzeitige Hautalterung und Faltenbildung. Von besonderer Bedeutung ist dabei der Wellenlängenbereich von 280 bis 400 nm. Dieser Bereich umfasst UV-B-Strahlen mit einer Wellenlänge zwischen 280 und 320 nm, die bei der Bildung eines Sonnenerythems eine entscheidende Rollen spielen, wie auch UV-A-Strahlen, mit einer Wellenlänge zwischen 320 und 400 nm, welche die Haut bräunen aber auch altem lassen, die Auslösung einer erythematösen Reaktion begünstigen oder diese Reaktion bei bestimmten Menschen vergrößern oder sogar phototoxische oder photoallergische und irritative Reaktionen auslösen können.

Hautschädigungen werden nicht nur durch Sonnenlicht verursacht, sondern auch durch andere äußere Einflüsse, wie Kälte oder Wärme. Ferner unterliegt die Haut einer natürlichen Alterung, wodurch Falten entstehen und die Spannkraft der Haut nachlässt.

Eine weitere Schwierigkeit bei der Herstellung von Kosmetika besteht darin, dass Wirkstoffe, die in kosmetische Zubereitungen eingearbeitet werden sollen, oftmals nicht stabil sind und in der Zubereitung geschädigt werden können. Die Schädigungen können beispielsweise durch eine Reaktion mit Luftsauerstoff oder durch die Absorption von UV-Strahlen verursacht werden. Die so geschädigten Moleküle können durch ihre Strukturänderung z.B. ihre Farbe ändern und/oder ihre Wirksamkeit verlieren.

Ein bekannter Weg, die beschriebenen Probleme zu behandeln besteht im Zusatz von Antioxidantien zu den Zubereitungen. Laut CD Römpp Chemie Lexikon - Version 1.0, Stuttgart/New York: Georg Thieme Verlag 1995 handelt es sich bei Antioxidantien um Verbindungen, die unerwünschte, durch Sauerstoff-Einwirkungen u.a. oxidative Prozesse bedingte Veränderungen in den zu schützenden Stoffen hemmen oder verhindern. Einsatzgebiete sind z.B. in Kunststoffen und Kautschuk zum Schutz gegen Alterung; in Fetten zum Schutz vor Ranzigkeit, in Ölen, Viehfutter, Autobenzin und Düsentreibstoffen zum Schutz gegen Verharzung, in Transformatoren- und Turbinenöl gegen Schlammbildung, in Aromastoffen gegen Geruchsverschlechterung. Als Antioxidantien wirksam sind u.a. durch sterisch hindernde Gruppen substituierte Phenole, Hydrochinone, Brenzcatechine und Aromat. Amine sowie deren MetallKomplexe. Die Wirkung der Antioxidantien besteht laut Römpp meist darin, daß sie als Radikalfänger für die bei der Autoxidation auftretenden freien Radikale wirken.

Es besteht daher weiterhin Bedarf nach hautverträglichen Antioxidantien, die sich auch zum Einsatz in hautpflegenden Zubereitungen eignen und sich in geeigneter Weise in kosmetische Zubereitungen einarbeiten lassen.

Aufgabe der Erfindung ist es daher, Verbindungen zur Verfügung zu stellen, welche in kosmetischen Zubereitungen appliziert eine schützende Wirkung gegen oxidativen Stress auf Körperzellen ausüben und/oder einer Alterung der Haut entgegenwirken können.

Überraschend wurde dabei gefunden dass [(4-Oxo-4H-chromen-3-yl)-hydroxymethyl]- oder [(4-Oxo-4H-chromen-3-yl)-methyl-phosphonsäure, deren Derivate oder deren Salze sich hervorragend als Antioxidantien auf der menschlichen Haut eignen.

Bisher sind unsubstituierte Chromon-3-methanphosphonsäuren und deren Methyl-, Ethyl-, Isopropyl- und n-Butylester bekannt. In Kostka et al, Phosphorus, Sulfur and Silicon and the related elements (1991), 57 (3-4), 279-85 wird jedoch lediglich die Synthese dieser Verbindungen beschrieben.

JP 1023 7081 A offenbart chromanol-Phosphate als Antoixidantien.

Ein erster Gegenstand der vorliegenden Erfindung sind daher Verbindungen der Formel I

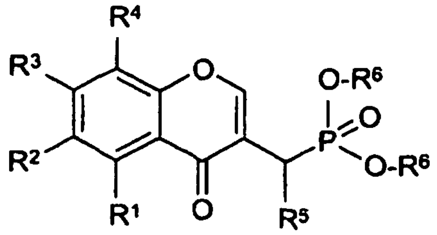

I

,

wobei
$R^1$ bis $R^3$ jeweils unabhängig voneinander H, Hydroxy oder Alkoxy mit 1 bis 8 C-Atomen bedeuten,
$R^4$ Alkyl mit 1 bis 4 C-Atomen, H, Hydroxy oder Alkoxy mit 1 bis 8 C-Atomen bedeutet,
$R^5$ H oder Hydroxy bedeutet und
$R^6$ H oder Alkyl mit 1 bis 18 C-Atomen bedeutet
oder deren Salze, wobei jedoch nicht alle $R^1$ bis $R^4$ zusammen gleich H sein dürfen.

In Formel I bedeutet Alkoxy mit 1 bis 8 C-Atomen beispielsweise Methoxy, Ethoxy, Propoxy, Butoxy, Pentoxy, Hexoxy, Heptoxy oder Octoxy, wobei jeweils auch die isomeren Vertreter der Alkylgruppen über ein Sauerstoff gebunden sein können.

In Formel I bedeutet Alkyl mit 1 bis 18 C-Atomen beispielsweise Methyl, Ethyl, n-Propyl, Isopropyl, n-Butyl, sek.-Butyl, tert-Butyl, n-$C_5H_{11}$ bis n-$C_{18}H_{37}$ und deren isomere Formen. Alkyl mit 1 bis 5 C-Atomen ist beispielsweise Methyl, Ethyl, n-Propyl, Isopropyl, n-Butyl, sek-Butyl, tert-Butyl und n-Pentyl.

Bevorzugt sind $R^1$ bis $R^3$ jeweils unabhängig voneinander H, Hydroxy, Methoxy oder Ethoxy, besonders bevorzugt H oder Hydroxy.
$R^4$ ist bevorzugt H, Methyl, Ethyl oder Hydroxy, besonders bevorzugt Methyl oder Hydroxy.

$R^1$ ist ganz besonders bevorzugt H.
$R^2$ und $R^3$ sind jeweils unabhängig voneinander ganz besonders bevorzugt Hydroxy.
$R^5$ ist ganz besonders bevorzugt Hydroxy.
$R^6$ ist besonders bevorzugt H oder Alkyl mit 1 bis 5 C-Atomen, ganz besonders bevorzugt H oder Methyl.

Ein weiterer Gegenstand der vorliegenden Erfindung ist entsprechend der bevorzugten Verwendung der erfindungsgemäßen Verbindungen eine Zubereitung, enthaltend zumindest eine Verbindung der Formel I.

Bei den Salzen handelt es sich vorzugsweise um solche mit Gegenionen, welche die Einarbeitung der Verbindungen der Formel I in Zubereitungen nicht behindern. Beispielsweise ist es bevorzugt, wenn es sich um Alkalimetall, Erdalkalimetall- oder Ammonium-Salze handelt. Insbesondere bevorzugt ist es erfindungsgemäß, wenn es sich bei den Salzen um Natrium-, Kalium-, Magnesium oder Ammonium-Salze, beispielsweise Triethanol-ammonium-Salze, handelt.

Vorteile der erfindungsgemäßen Verbindungen bzw. Zusammensetzungen sind dabei insbesondere die antioxidante Wirkung und die gute Hautverträglichkeit. Von Vorteil ist insbesondere das besondere Wirkprofil der erfindungsgemäß einzusetzenden Verbindungen, welches sich durch Stabilität der Verbindungen in den Zubereitungen und antioxidante Wirkung in oder auf der Haut äußert.

Ein Gegenstand der vorliegenden Erfindung ist aufgrund dieser Eigenschaften die Verwendung der Verbindungen gemäß Formel I, wie oben angegeben, zur Herstellung einer Zubereitung, insbesondere mit antioxidanten Eigenschaften bzw. zur Herstellung von Zubereitungen, die eine schützende Wirkung gegen oxidativen Stress auf Körperzellen ausüben und/oder einer Alterung der Haut entgegenwirken bzw. zur Minderung der Folgen der Hautalterung beitragen können.

Bei den Zubereitungen handelt es sich dabei üblicherweise entweder um topisch anwendbare Zubereitungen, beispielsweise kosmetische, pharmazeutische oder dermatologische Formulierungen, oder um Nahrungsmittel bzw. Nahrungsergänzungsmittel. Die Zubereitungen enthalten in diesem Fall einen kosmetisch, pharmazeutisch oder dermatologisch oder Nahrungsmittel-geeigneten Träger und je nach gewünschtem Eigenschaftsprofil optional weitere geeignete Inhaltsstoffe. Bevorzugt werden die topischen Zubereitungen als kosmetische oder dermatologische Zubereitung eingesetzt.

Im Sinne der vorliegenden Erfindung wird der Begriff Zubereitung oder Formulierung gleichbedeutend verwendet.

Die Verbindungen der Formel I werden erfindungsgemäß typisch in Mengen von 0,01 Gew.-% bis 20 Gew.-%, vorzugsweise in Mengen von 0,1 Gew.-% bis 10 Gew.-% und insbesondere bevorzugt in Mengen von 1 Gew.-% bis 8 Gew.-% eingesetzt. Dabei bereitet es dem Fachmann keinerlei Schwierigkeiten die Mengen abhängig von der beabsichtigten Wirkung der Zubereitung entsprechend auszuwählen.

Damit die Verbindungen der Formel I ihre positive Wirkung als Radikalfänger auf die Haut besonders gut entwickeln können, kann es bevorzugt sein die Verbindungen der Formel I in tiefere Hautschichten eindringen zu lassen. Dazu stehen mehrere Möglichkeiten zur Verfügung, beispielsweise können in der Zubereitung auch entsprechende Transportmittel, beispielsweise Liposomen, vorgesehen sein, die einen Transport der Verbindungen der Formel I durch die äußeren Hautschichten ermöglichen. Schließlich ist auch ein systemischer Transport der Verbindungen der Formel I denkbar. Die Zubereitung wird dann beispielsweise so gestaltet, dass sie für eine orale Gabe geeignet ist.

Allgemein wirken die Substanzen der Formel I als Radikalfänger. Solche Radikale werden nicht nur durch Sonnenlicht erzeugt, sondern werden unter verschiedenen Bedingungen gebildet. Beispiele sind Anoxie, die den Elektronenfluß stromauf der Cytochromoxidasen blockiert und die Bildung von Superoxidradikalanionen bedingt; Entzündungen, die unter anderem mit der Bildung von Superoxidanionen durch die Membran-NADPH-Oxidase der Leukozyten einhergehen, die jedoch auch mit der Bildung (durch Disproportionierung in Gegenwart von Eisen (II)-ionen) der Hydroxyradikale und anderer reaktiver Spezies, die normalerweise beim Phänomen einer Phagocytose beteiligt sind, einhergehen; sowie Lipidautooxidation die im Allgemeinen durch ein Hydroxylradikal initiiert wird und lipidische Alkoxyradikale und Hydroperoxide liefert.

Es wird vermutet, dass bevorzugte Verbindungen der Formel I auch als Enzymhemmer wirken. Sie hemmen vermutlich Histidindecarboxylase, Proteinkinasen, Elastase, Aldosereduktase sowie Hyaluronidase, und ermöglichen daher, die Unversehrtheit der Grundsubstanz vaskulärer Hüllen aufrecht zu erhalten. Ferner hemmen sie vermutlich nicht spezifisch Katechol-O-methyltransferase, wodurch die Menge der verfügbaren Katecholamine und dadurch die Gefäßfestigkeit erhöht wird. Weiter hemmen sie AMP-Phosphodiesterase, wodurch die Substanzen ein Potential zur Hemmung der Thrombozytenaggregation aufweisen.
Aufgrund dieser Eigenschaften eignen sich die erfindungsgemäßen Verbindungen oder Zubereitungen allgemein zur Immunprotektion und zum Schutz der DNA und RNA. Insbesondere eignen sich die Zubereitungen dabei zum Schutz von DNA und RNA vor oxidativen Angriffen, vor Radikalen und vor Schädigung durch Strahlung, insbesondere UV-Strahlung. Ein weiterer Vorteil der erfindungsgemäßen Zubereitungen ist der Zellschutz, insbesondere der Schutz von Langerhans-Zellen vor Schäden durch die oben genannten Einflüsse. Alle diese Verwendungen bzw. die Verwendung der Verbindungen der Formel I zur Herstellung entsprechend einsetzbarer Zubereitungen sind möglich.

Insbesondere eignen sich bevorzugte erfindungsgemäße Zubereitungen auch zur Behandlung von Hautkrankheiten, die mit einer Störung der Keratinisierung verbunden sind, die die Differenzierung und Zellproliferation betrifft, insbesondere zur Behandlung der Akne vulgaris, Akne comedonicá, der polymorphen Akne, der Akne rosaceae, der nodulären Akne, der Akne conglobata, der alters-bedingten Aknen, der als Neben-wirkung auftretenden Aknen, wie der Akne solaris, der medikamenten-bedingten Akne oder der Akne professionalis, zur Behandlung anderer Störungen der Keratinisierung, insbesondere der Ichtyosen, der ichtyosi-formen Zustände, der Darrier-Krankheit, der Keratosis palmoplantaris, der Leukoplasien, der leukoplasiformen Zustände, der Haut- und Schleimhaut-flechten (Buccal) (Lichen), zur Behandlung anderer Hauterkrankungen, die mit einer Störung der Keratinisierung zusammenhängen und eine entzündliche und/oder immunoallergische Komponente haben und insbesondere aller Formen der Psoriasis, die die Haut, die Schleimhäute und die Finger und Zehennägel betreffen, und des psoriatischen Rheumas und der Haut-atopien, wie Ekzemen oder der respiratorischen Atopie oder auch der Hypertrophie des Zahnfleisches, wobei die Verbindungen ferner bei einigen Entzündungen verwendet werden können, die nicht mit einer Störung der Keratinisierung zusammenhängen, zur Behandlung aller gutartigen oder bösartigen Wucherungen der Dermis oder Epidermis, die gegebenenfalls viralen Ursprungs sind, wie Verruca vulgaris. Veruca plana, Epidermodysplasia verruciformis, orale Papillomatose, Papillo-matosis florida, und der Wucherungen, die durch UV-Strahlung hervor-gerufen werden können, insbesondere des Epithelioma baso-cellulare und Epithelioma spinocellulare, zur Behandlung anderer Hautkrankheiten, wie der Dermatitis bullosa und der das Kollagen betreffenden Krankheiten, zur Behandlung bestimmter Augenkrankheiten, insbesondere der Hornhauterkrankungen, zur Behebung oder Bekämpfung der lichtbedingten und der mit dem Älterwerden zusammenhängenden Hautalterung, zur Verminderung der Pigmentierungen und der Keratosis actinica und zur Behandlung aller Krankheiten, die mit der normalen Alterung oder der lichtbedingten Alterung zusammenhängen, zur Vorbeugung vor oder der Heilung von Wunden/Narben der Atrophien der Epidermis und/oder Dermis, die durch lokal oder systemisch angewendete Corticosteroide hervorgerufen werden und aller sonstigen Arten der Hautatrophie, zur Vorbeugung vor oder Behandlung von Störungen der Wundheilung, zur Vermeidung oder Behebung von Schwangerschaftsstreifen oder auch zur Förderung der Wundheilung, zur Bekämpfung von Störungen der Talgproduktion, wie Hypersebhorrhö bei Akne oder der einfachen Seborrhö, zur Bekämpfung von oder Vorbeugung von krebsartigen Zuständen oder vor präkanzerogenen Zuständen, insbesondere der promyelozytären Leukämien, zur Behandlung von Entzündungserkrankungen, wie Arthritis, zur Behandlung aller virusbedingten Erkrankungen der Haut oder anderer Bereiche des Körpers, zur Vorbeugung vor oder Behandlung der Alopecie, zur Behandlung von Hautkrankheiten oder Krankheiten anderer Körperbereiche mit einer immunologischen Komponente, zur Behandlung von Herz-/Kreislauf-Erkrankungen, wie Arteriosklerose oder Bluthochdruck, sowie des Insulin-unabhängigen Diabetes, zur Behandlung von Hautproblemen, die durch UV-Strahlung hervorgerufen werden.

Die antioxidativen Wirkungen der Verbindungen gemäß Formel I können beispielsweise mit dem 2,2-Diphenyl-1-picrylhydrazyl(DPPH)-Assay gezeigt werden. 2,2-Diphenyl-1-picrylhydrazyl ist ein in Lösung stabiles freies Radikal. Das ungepaarte Elektron führt zu einer starken Absorptionsbande bei 515 nm, die Lösung ist dunkel-violett gefärbt. In Gegenwart eines Radikalfängers wird das Elektron gepaart, die Absorption verschwindet und die Entfärbung verläuft stöchiometrisch unter Berücksichtigung der aufgenommenen Elektronen. Gemessen wird die Extinktion im Photometer. Die antiradikalische Eigenschaft der zu testenden Substanz wird bestimmt, indem man die Konzentration ermittelt, bei der 50 % des eingesetzten 2,2-Diphenyl-1-picrylhydrazyls mit dem Radikalfänger reagiert haben. Ausgedrückt wird diese Konzentration als $EC_{50}$, ein Wert, der unter den gegebenen Messbedingungen als Substanzeigenschaft zu betrachten ist. Verglichen wird die untersuchte Substanz mit einem Standard (z.B. Tocopherol). Der $EC_{50}$-Wert ist dabei ein Maß für die Kapazität der jeweiligen Verbindung Radikale zu fangen. Je niedriger der $EC_{50}$-Wert ist, desto höher ist die Kapazität Radikale zu fangen. Ein weiterer wichtiger Aspekt für die Wirkung der Antioxidantien ist die Zeit in der dieser $EC_{50}$-Wert erreicht wird. Diese Zeit gemessen in Minuten ergibt den $T_{EC50}$-Wert, der eine Aussage über die Geschwindigkeit zulässt, mit der diese Antioxidantien Radikale fangen. Im Sinne dieser Erfindungen gelten Antioxidantien, die diesen Wert innerhalb von weniger als 60 Minuten erreichen als schnell, solche die den $EC_{50}$-Wert erst nach mehr als 120 Minuten erreichen als zeitverzögert wirkend.

Die antiradikalische Effizienz (AE) (beschrieben bei C. Sanchez-Moreno, J.A. Larrauri und F. Saura-Calixto in J. Sci. Food Agric. 1998, 76(2), 270-276.) ergibt sich aus den oben genannten Größen nach folgender Beziehung:

$$AE = \frac{1}{EC_{50} T_{EC50}}$$

Eine niedrige AE ($x10^{-3}$) liegt im Bereich bis etwa 10, von einer mittleren AE wird im Bereich von 10 bis 20 gesprochen und eine hohe AE liegt erfindungsgemäß bei Werten oberhalb 20 vor.

Dabei kann es erfindungsgemäß insbesondere bevorzugt sein, schnell wirkende Antioxidantien mit solchen mit langsamer oder zeitverzögerter Wirkung zu kombinieren. Dabei sind typische Gewichtsprozentverhältnisse der schnell wirkenden Antioxidantien zu zeitverzögert wirkenden Antioxidantien im Bereich 10:1 bis 1:10, vorzugsweise im Bereich 10:1 bis 1:1 und für hautschützende Zubereitungen insbesondere bevorzugt im Bereich 5:1 bis 2:1. In anderen erfindungsgemäß ebenfalls bevorzugten Zubereitungen kann es im Sinne einer Wirkungsoptimierung allerdings von Vorteil sein, mehr zeitverzögert wirkende Antioxidantien als schnell wirkende Antioxidanten vorliegen. Typische Zusammensetzungen zeigen dann Gewichtsprozentverhältnisse der schnell wirkenden Antioxidantien zu zeitverzögert wirkenden Antioxidantien im Bereich 1:1 bis 1:10, vorzugsweise im Bereich 1:2 bis 1:8.

Die schützende Wirkung gegen oxidativen Stress bzw. gegen die Einwirkung von Radikalen kann also weiter verbessert werden, wenn die Zubereitungen ein oder mehrere weitere Antioxidantien enthalten, wobei es dem Fachmann keinerlei Schwierigkeiten bereitet geeignet schnell oder zeitverzögert wirkende Antioxidantien auszuwählen.

In einer bevorzugten Ausführungsform der vorliegenden Erfindungen handelt es sich bei der Zubereitung daher um eine Zubereitung zum Schutz von Körperzellen gegen oxidativen Stress, insbesondere zur Verringerung der Hautalterung, **dadurch gekennzeichnet, dass** sie neben den ein oder mehreren Verbindungen nach Formel I vorzugsweise ein oder mehrere weitere Antioxidantien enthält.

Es gibt viele aus der Fachliteratur bekannte und bewährte Substanzen, die als Antioxidantien verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Vitamin A und Derivate (z.B. Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (z.B. ZnO, $ZnSO_4$), Selen und dessen Derivate (z.B. Selenmethionin), Stilbene und deren Derivate (z.B. Stilbenoxid, trans-Stilbenoxid).

Geeignete Antioxidantien sind auch Verbindungen der allgemeinen Formeln A oder B

A 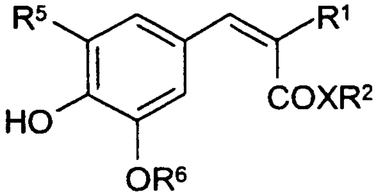 oder 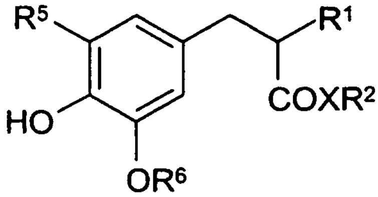 B ,

worin
$R^1$ aus der Gruppe $-C(O)CH_3$, $-CO_2R^3$, $-C(O)NH_2$ und $-C(O)N(R^4)_2$ ausgewählt werden kann,
X O oder NH,
$R^2$ lineares oder verzweigtes Alkyl mit 1 bis 30 C-Atomen,
$R^3$ lineares oder verzweigtes Alkyl mit 1 bis 20 C-Atomen,
$R^4$ jeweils ungabhängig voneinander H oder lineares oder verzweigtes Alkyl mit 1 bis 8 C-Atomen,
$R^5$ lineares oder verzweigtes Alkyl mit 1 bis 8 C-Atomen oder lineares oder verzweigtes Alkoxy mit 1 bis 8 C-Atomen und
$R^6$ lineares oder verzweigtes Alkyl mit 1 bis 8 C-Atomen bedeutet, vorzugsweise Derivate der 2-(4-Hydroxy-3,5-dimethoxybenzyliden)-malonsäure und/oder 2-(4-Hydroxy-3,5-dimethoxybenzyl)-malonsäure, besonders bevorzugt 2-(4-Hydroxy-3,5-dimethoxybenzyliden)-malonsäure-bis-(2-ethylhexyl)ester (z.B. Oxynex® ST Liquid) und/oder 2-(4-Hydroxy-3,5-dimethoxybenzy)-malonsäure-bis-(2-ethylhexyl)ester (z.B. RonaCare® AP).

Mischungen von Antioxidantien sind ebenfalls zur Verwendung in den erfindungsgemäßen kosmetischen Zubereitungen geeignet. Bekannte und käufliche Mischungen sind beispielsweise Mischungen enthaltend als aktive Inhaltsstoffe Lecithin, L-(+)-Ascorbylpalmitat und Zitronensäure (z.B. (z.B. Oxynex® AP), natürliche Tocopherole, L-(+)-Ascorbylpalmitat, L-(+)-Ascorbinsäure und Zitronensäure (z.B. Oxynex® K LIQUID), Tocopherolextrakte aus natürlichen Quellen, L-(+)-Ascorbylpalmitat, L-(+)-Ascorbinsäure und Zitronensäure (z.B. Oxynex® L LIQUID), DL-α-Tocopherol, L-(+)-Ascorbylpalmitat, Zitronensäure und Lecithin (z.B. Oxynex® LM) oder Butylhydroxytoluol (BHT), L-(+)-Ascorbylpalmitat und Zitronensäure (z.B. Oxynex® 2004). Derartige Antioxidantien werden mit Verbindungen der Formell I in solchen Zusammensetzungen überlicherweise in Gewichtsprozentverhältnissen im Bereich von 1000:1 bis 1:1000, bevorzugt in Gewichtsprozentverhältnissen von 100:1 bis 1:100 eingesetzt.

Die erfindungsgemäßen Zubereitungen können als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin $B_1$), Riboflavin (Vitamin $B_2$), Nicotinsäureamid, Vitamin C (Ascorbinsäure), Vitamin D, Ergocalciferol (Vitamin $D_2$), Vitamin E, DL-α-Tocopherol, Tocopherol-E-Acetat, Tocopherolhydrogensuccinat, Vitamin $K_1$, Esculin (Vitamin P-Wirkstoff), Thiamin (Vitamin $B_1$), Nicotinsäure (Niacin), Pyridoxin, Pyridoxal, Pyridoxamin, (Vitamin $B_6$), Panthothensäure, Biotin, Folsäure und Cobalamin (Vitamin $B_{12}$) in den erfindungsgemäßen kosmetischen Zubereitungen enthalten, insbesondere bevorzugt Vitamin-A-Palmitat, Vitamin C und dessen Derivaten, DL-α-Tocopherol, Tocopherol-E-Acetat, Nicotinsäure, Pantothensäure und Biotin. Vitamine werden dabei mit Verbindungen der Formel I überlicherweise in Gewichtsprozentverhältnissen im Bereich von 1000:1 bis 1:1000, bevorzugt in Gewichtsprozentverhältnissen von 100:1 bis 1:100 eingesetzt.

Erfindungsgemäß insbesondere bevorzugte Zubereitungen enthalten neben den Verbindungen der Formel I auch reine UV-Filter.

Bei Einsatz der als UV-A-Filter insbesondere bevorzugten Dibenzoylmethanderivate in Kombination mit den Verbindungen der Formel I ergibt sich ein zusätzlicher Vorteil: Die UV-empfindlichen Dibenzoylmethanderivate werden durch die Anwesenheit der Verbindungen der Formel I zusätzlich stabilisiert. Ein weiterer Gegenstand der vorliegenden Erfindung ist daher die Verwendung der Verbindungen gemäß Formel I zur Stabilisierung von Dibenzoylmethanderivaten in Zubereitungen.

Prinzipiell kommen alle UV-Filter für eine Kombination mit den erfindungsgemäßen Verbindungen der Formel I in Frage. Besonders bevorzugt sind solche UV-Filter, deren physiologische Unbedenklichkeit bereits nachgewiesen ist. Sowohl für UVA wie auch UVB-Filter gibt es viele aus der Fachliteratur bekannte und bewährte Substanzen, z.B.

Benzylidenkampferderivate wie 3-(4'-Methylbenzyliden)-dl-kampfer (z.B. Eusolex® 6300), 3-Benzylidenkampfer (z.B. Mexoryl® SD), Polymere von N-{(2 und 4)-[(2-oxoborn-3-yliden)methyl]benzyl}-acrylamid (z.B. Mexoryl® SW), N,N,N-Trimethyl-4-(2-oxoborn-3-ylidenmethyl)anilinium methylsulfat (z.B. Mexoryl® SK) oder (2-Oxoborn-3-yliden)toluol-4-sulfonsäure (z.B. Mexoryl® SL),

Benzoyl- oder Dibenzoylmethane wie 1-(4-tert-Butylphenyl)-3-(4-methoxyphenyl)propan-1,3-dion (z.B. Eusolex® 9020) oder 4-Isopropyldibenzoylmethan (z.B. Eusolex® 8020),
Benzophenone wie 2-Hydroxy-4-methoxybenzophenon (z.B. Eusolex® 4360) oder 2-Hydroxy-4-methoxybenzophenon-5-sulfonsäure und ihr Natriumsalz (z.B. Uvinul® MS-40), Methoxyzimtsäureester wie Methoxyzimtsäureoctylester (z.B. Eusolex® 2292), 4-Methoxyzimtsäureisopentylester, z.B. als Gemisch der Isomere (z.B. Neo Heliopan® E 1000), Salicylatderivate wie 2-Ethylhexylsalicylat (z.B. Eusolex® OS), 4-Isopropylbenzylsalicylat (z.B. Megasol®) oder 3,3,5-Trimethylcyclohexylsalicylat (z.B. Eusolex® HMS), 4-Aminobenzoesäure und Derivate wie 4-Aminobenzoesäure, 4-(Dimethylamino)benzoesäure-2-ethylhexylester (z.B. Eusolex® 6007), ethoxylierter 4-Aminobenzoesäureethylester (z.B. Uvinul® P25), Phenylbenzimidazolsulfonsäuren, wie 2-Phenylbenzimidazol-5-sulfonsäure sowie ihre Kalium-, Natrium- und Triethanolaminsalze (z.B. Eusolex® 232), 2,2-(1,4-Phenylen)-bisbenzimidazol-4,6-disulfonsäure bzw. deren Salze (z.B. Neoheliopan® AP) oder 2,2-(1,4-Phenylen)-bisbenzimidazol-6-sulfonsäure; und weitere Substanzen wie

- 2-Cyano-3,3-diphenylacrylsäure-2-ethylhexylester (z.B. Eusolex® OCR),
- 3,3'-(1,4-Phenylendimethylen)-bis-(7,7-dimethyl-2-oxobicyclo-[2.2.1]hept-1-ylmethansulfonsäure sowie ihre Salze (z.B. Mexoryl® SX) und
- 2,4,6-Trianilino-(p-carbo-2'-ethylhexyl-1'-oxi)-1,3,5-triazin ( z.B. Uvinul® T 150)
- 2-(4-Diethylamino-2-hydroxy-benzoyl)-benzoesäure hexylester (z.B. Uvinul®UVA Plus, Fa. BASF).

Die in der Liste aufgeführten Verbindungen sind nur als Beispiele aufzufassen. Selbstverständlich können auch andere UV-Filter verwendet werden.

Diese organischen UV-Filter werden in der Regel in einer Menge von 0,5 bis 10 Gewichtsprozent, vorzugsweise 1 - 8 Gew.-%, in kosmetische Formulierungen eingearbeitet.

Weitere geeignete organische UV-Filter sind z.B.

- 2-(2H-Benzotriazol-2-yl)-4-methyl-6-(2-methyl-3-(1,3,3,3-tetramethyl-1-(trimethylsilyloxy)disiloxanyl)propyl)phenol (z.B. Silatrizole®),
- α-(Trimethylsilyl)-ω-[trimethylsilyl)oxy]poly[oxy(dimethyl [und ca. 6% methyl[2-[p-[2,2-bis(ethoxycarbonyl]vinyl] phenoxy]-1-methylenethyl] und ca. 1,5 % methyl[3-[p-[2,2-bis(ethoxycarbonyl)vinyl)phenoxy)-propenyl) und 0,1 bis 0,4% (methylhydrogen]silylenl] (n ≈ 60) (CAS-Nr. 207 574-74-1)
- 2,2'-Methylen-bis-(6-(2H-benzotriazol-2-yl)-4-(1,1,3,3-tetramethylbutyl)phenol) (CAS-Nr. 103 597-45-1)
- 2,2'-(1,4-Phenylen)bis-(1H-benzimidazol-4,6-disulfonsäure, Mononatriumsalz) (CAS-Nr. 180 898-37-7) und
- 2,4-bis-{[4-(2-Ethyl-hexyloxy)-2-hydroxyl]-phenyl}-6-(4-methoxyphenyl)-1,3,5-triazin (CAS-Nr. 103 597-45-, 187 393-00-6).
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb® HEB).

Organische UV-Filter werden in der Regel in einer Menge von 0,5 bis 20 Gewichtsprozent, vorzugsweise 1 - 15 Gew.-%, in kosmetische Formulierungen eingearbeitet.

Als anorganische UV-Filter sind solche aus der Gruppe der Titandioxide wie z.B. gecoatetes Titandioxid (z.B. Eusolex® T-2000, Eusolex®T-AQUA), Zinkoxide (z.B. Sachtotec®), Eisenoxide oder auch Ceroxide denkbar. Diese anorganischen UV-Filter werden in der Regel in einer Menge von 0,5 bis 20 Gewichtsprozent, vorzugsweise 2 - 10 Gew.-%, in kosmetische Zubereitungen eingearbeitet.

Bevorzugte Verbindungen mit UV-filtemden Eigenschaften sind 3-(4'-Methylbenzyliden)-dl-kampfer, 1-(4-tert-Butylphenyl)-3-(4-methoxy-phenyl)-pro-pan-1,3-dion, 4-Isopropyldibenzoylmethan, 2-Hydroxy-4-meth-oxy-ben-zo--phenon, Methoxyzimtsäureoctylester, 3,3,5-Trimethyl-cyclo-hexylsali-cylat, 4-(Dimethylamino)benzoesäure-2-ethyl-hexylester, 2-Cyano-3,3-di-phenyl-acrylsäure-2-ethylhexylester, 2-Phenyl-benzimidazol-5-sulfonsäure sowie ihre Kalium-, Natrium- und Triethanol-aminsalze.

Durch Kombination von einer oder mehrerer Verbindungen der Formel I mit weiteren UV-Filtern kann die Schutzwirkung gegen schädliche Einwirkungen der UV-Strahlung optimiert werden.

Optimierte Zusammensetzungen können beispielsweise die Kombination der organischen UV-Filter 4'-Methoxy-6-hydroxyflavon mit 1-(4-tert-Butylphenyl)-3-(4-methoxyphenyl)propan-1,3-dion und 3-(4'-Methylbenzyliden)-dl-kampfer enthalten. Mit dieser Kombination ergibt sich ein Breitbandschutz, der durch Zusatz von anorganischen UV-Filtern, wie Titandioxid-Mikropartikeln noch ergänzt werden kann.

Alle genannten UV-Filter können auch in verkapselter Form eingesetzt werden. Insbesondere ist es von Vorteil organische UV-Filter in verkapselter Form einzusetzen. Im Einzelnen ergeben sich die folgende Vorteile:

- Die Hydrophilie der Kapselwand kann unabhängig von der Löslichkeit des UV-Filters eingestellt werden. So können beispielsweise auch hydrophobe UV-Filter in rein wässrige Zubereitungen eingearbeitet werden. Zudem wird der

häufig als unangenehm empfundene ölige Eindruck beim Auftragen der hydrophobe UV-Filter enthaltenden Zubereitung unterbunden.

- Bestimmte UV-Filter, insbesondere Dibenzoylmethanderivate, zeigen in kosmetischen Zubereitungen nur eine verminderte Photostabilität. Durch Verkapselung dieser Filter oder von Verbindungen, die die Photostabilität dieser Filter beeinträchtigen, wie beispielsweise Zimtsäurederivate, kann die Photostabilität der gesamten Zubereitung erhöht werden.
- In der Literatur wird immer wieder die Hautpenetration durch organische UV-Filter und das damit verbundene Reizpotential beim direkten Auftragen auf die menschliche Haut diskutiert. Durch die hier vorgeschlagene Verkapselung der entsprechenden Substanzen wird dieser Effekt unterbunden.
- Allgemein können durch Verkapselung einzelner UV-Filter oder anderer Inhaltstoffe Zubereitungsprobleme, die durch Wechselwirkung einzelner Zubereitungsbestandteile untereinander entstehen, wie Kristallisationsvorgänge, Ausfällungen und Agglomeratbildung vermieden werden, da die Wechselwirkung unterbunden wird.

Daher ist es erfindungsgemäß bevorzugt, wenn ein oder mehrere der oben genannten UV-Filter in verkapselter Form vorliegen. Vorteilhaft ist es dabei, wenn die Kapseln so klein sind, dass sie mit dem bloßen Auge nicht beobachtet werden können. Zur Erzielung der o.g. Effekte ist es weiterhin erforderlich, dass die Kapseln hinreichend stabil sind und den verkapselten Wirkstoff (UV-Filter) nicht oder nur in geringem Umfang an die Umgebung abgeben.

Geeignete Kapseln können Wände aus anorganischen oder organischen Polymeren aufweisen. Beispielsweise wird in US 6,242,099 B1 die Herstellung geeigneter Kapseln mit Wänden aus Chitin, Chitin-Derivaten oder polyhydroxylierten Polyaminen beschrieben. Erfindungsgemäß besonders bevorzugt einzusetzende Kapseln weisen Wände auf, die durch einen SolGel-Prozeß, wie er in den Anmeldungen WO 00/09652, WO 00/72806 und WO 00/71084 beschrieben ist, erhalten werden können. Bevorzugt sind hier wiederum Kapseln, deren Wände aus Kieselgel (Silica; undefiniertes Silicium-oxid-hydroxid) aufgebaut sind. Die Herstellung entsprechender Kapseln ist dem Fachmann beispielsweise aus den zitierten Patentanmeldungen bekannt, deren Inhalt ausdrücklich auch zum Gegenstand der vorliegenden Anmeldung gehört.

Dabei sind die Kapseln in erfindungsgemäßen Zubereitungen vorzugsweise in solchen Mengen enthalten, die gewährleisten, dass die verkapselten UV-Filter in den oben angegebenen Mengen in der Zubereitung vorliegen.

Die erfindungsgemäßen Zubereitungen können darüber hinaus weitere übliche hautschonende oder hautpflegende Wirkstoffe enthalten. Dies können prinzipiell alle den Fachmann bekannten Wirkstoffe sein.

Besonders bevorzugte Wirkstoffe sind Pyrimidincarbonsäuren und/oder Aryloxime.

Pyrimidincarbonsäuren kommen in halophilen Mikroorganismen vor und spielen bei der Osmoregulation dieser Organismen eine Rolle (E. A. Galinski et al., Eur. J. Biochem., 149 (1985) Seite 135-139). Dabei sind unter den Pyrimidincarbonsäuren insbesondere Ectoin ((S)-1,4,5,6-Tetrahydro-2-methyl-4-pyrimidincarbonsäure) und Hydroxyectoin ((S,S)-1,4,5,6-Tetrahydro-5-hydroxy-2-methyl-4-pyrimidincarbonsäure und deren Derivate zu nennen. Diese Verbindungen stabilisieren Enzyme und andere Biomoleküle in wässrigen Lösungen und organischen Lösungsmitteln. Weiter stabilisieren sie insbesondere Enzyme gegen denaturierende Bedingungen, wie Salze, extreme pH-Werte, Tenside, Harnstoff, Guanidiniumchlorid und andere Verbindungen.

Ectoin und Ectoin-Derivate wie Hydroxyectoin können vorteilhaft in Arzneimitteln verwendet werden. Insbesondere kann Hydroxyectoin zur Herstellung eines Arzneimittels zur Behandlung von Hauterkrankungen eingesetzt werden. Andere Einsatzgebiete des Hydroxyectoins und anderer Ectoin-Derivate liegen typischerweise in Gebieten in denen z.B. Trehalose als Zusatzstoff verwendet wird. So können Ectoin-Derivate, wie Hydroxyectoin, als Schutzstoff in getrockneten Hefe- und Bakterienzellen Verwendung finden. Auch pharmazeutische Produkte wie nicht glykosylierte, pharmazeutische wirksame Peptide und Proteine z.B. t-PA können mit Ectoin oder seinen Derivaten geschützt werden.

Unter den kosmetischen Anwendungen ist insbesondere die Verwendung von Ectoin und Ectoin-Derivaten zur Pflege von gealterter, trockener oder gereizter Haut zu nennen. So wird in der europäischen Patentanmeldung EP- A-0 671 161 insbesondere beschrieben, dass Ectoin und Hydroxyectoin in kosmetischen Zubereitungen wie Pudern, Seifen, tensidhaltigen Reinigungsprodukten, Lippenstiften, Rouge, Make-Ups, Pflegecremes und Sonnenschutzpräparaten eingesetzt werden.

Dabei wird vorzugsweise eine Pyrimidincarbonsäure gemäß der unten stehenden Formel C eingesetzt,

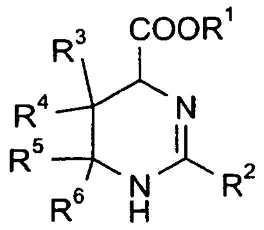

C

worin $R^1$ ein Rest H oder $C_{1-8}$-Alkyl, $R^2$ ein Rest H oder $C_{1-4}$-Alkyl und $R^3$, $R^4$, $R^5$ sowie $R^6$ jeweils unabhängig voneinander ein Rest aus der Gruppe H, OH, $NH_2$ und $C_{1-4}$-Alkyl sind. Bevorzugt werden Pyrimidincarbonsäuren eingesetzt, bei denen $R^2$ eine Methyl- oder eine Ethylgruppe ist und $R^1$ bzw. $R^5$ und $R^6$ H sind. Insbesondere bevorzugt werden die Pyrimidincarbonsäuren Ectoin ((S)-1,4,5,6-Tetrahydro-2-methyl-4-pyrimidin-carbonsäure) und Hydroxyectoin ((S, S)-1,4,5,6-Tetrahydro-5-hydroxy-2-methyl-4-pyrimidin-carbonsäure) eingesetzt. Dabei enthalten die erfindungsgemäßen Zubereitungen derartige Pyrimidincarbonsäuren vorzugsweise in Mengen bis zu 15 Gew.-%. Vorzugsweise werden die Pyrimidincarbonsäuren dabei in Gewichtsprozentverhältnissen von 100:1 bis 1:100 zu den Verbindungen der Formel I eingesetzt, wobei Gewichtsprozentverhältnisse im Bereich 1:10 bis 10:1 besonders bevorzugt sind.

Unter den Aryloximen wird vorzugsweise 2-Hydroxy-5-methyllaurophenonoxim, welches auch als HMLO, LPO oder F5 bezeichnet wird, eingesetzt. Seine Eignung zum Einsatz in kosmetischen Mitteln ist beispielsweise aus der Deutschen Offenlegungsschrift DE-A-41 16 123 bekannt. Zubereitungen, die 2-Hydroxy-5-methyllaurophenonoxim enthalten, sind demnach zur Behandlung von Hauterkrankungen, die mit Entzündungen einhergehen, geeignet. Es ist bekannt, dass derartige Zubereitungen z.B. zur Therapie der Psioriasis, unterschiedlicher Ekzemformen, irritativer und toxischer Dermatitis, UV-Dermatitis sowie weiterer allergischer und/oder entzündlicher Erkrankungen der Haut und der Hautanhangsgebilde verwendet werden können. Erfindungsgemäße Zubereitungen, die neben der Verbindung der Formel I zusätzlich eine Aryloxim, vorzugsweise 2-Hydroxy-5-methyllaurophenonoxim enthalten, zeigen überraschende antiinflammatorische Eignung. Dabei enthalten die Zubereitungen vorzugsweise 0,01 Gew.-% bis 10 Gew.-% des Aryloxims, wobei es insbesondere bevorzugt ist, wenn die Zubereitung 0,05 bis 5 Gew-% Aryloxim enthält.

Alle Verbindungen oder Komponenten, die in den Zubereitungen verwendet werden können, sind entweder bekannt und käuflich erwerbbar oder können nach bekannten Verfahren synthetisiert werden.

Die eine oder die mehreren Verbindungen der Formel I können in der üblichen Weise in kosmetische oder dermatologische Zubereitungen eingearbeitet werden. Geeignet sind Zubereitungen für eine äußerliche Anwendung, beispielsweise als Creme, Lotion, Gel, oder als Lösung, die auf die Haut aufgesprüht werden kann. Für eine innerliche Anwendung sind Darreichungsformeln wie Kapseln, Dragees, Pulver, Tabletten-Lösungen oder Lösungen geeignet.

Als Anwendungsform der erfindungsgemäßen Zubereitungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole und Sprays. Weitere Anwendungsformen sind z.B. Sticks, Shampoos und Duschbäder. Der Zubereitung können beliebige übliche Trägerstoffe, Hilfsstoffe und gegebenenfalls weitere Wirkstoffe zugesetzt werden.

Vorzuziehende Hilfsstoffe stammen aus der Gruppe der Konservierungsstoffe, Stabilisatoren, Lösungsvermittler, Färbemittel, Geruchsverbesserer. Salben, Pasten, Cremes und Gele können die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethlyacetat, Benzylalkohol, Benzylbenzoat, Propylenglykol, 1,3-Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobemsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typische kosmetische Anwendungsformen sind auch Lippenstifte, Lippenpflegestifte, Mascara, Eyeliner, Lidschatten, Rouge, Puder-, Emulsions- und Wachs-Make up sowie Sonnenschutz-, Prä-Sun- und After-Sun-Präparate.

Zu den bevorzugten erfindungsgemäßen Zubereitungsformen gehören insbesondere Emulsionen.

Erfindungsgemäße Emulsionen sind vorteilhaft und enthalten z. B. die genannten Fette, Öle, Wachse und anderen Fettkörper, sowie Wasser und einen Emulgator, wie er üblicherweise für einen solchen Typ der Zubereitung verwendet wird.

Die Lipidphase kann vorteilhaft gewählt werden aus folgender Substanzgruppe:

- Mineralöle, Mineralwachse
- Öle, wie Triglyceride der Caprin- oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fett-Ikoholen mit Alkansäuren niedriger C-Zahl oder mit Fettsäuren;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigtem und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäure und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexaldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12-18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylether.

Besonders vorteilhaft sind Mischungen aus $C_{12-15}$-Alkylbenzoat und 2-Ethylhexylisostearat, Mischungen aus $C_{12-15}$-Alkylbenzoat und Isotridecylisononanoat sowie Mischungen aus $C_{12-15}$-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann auch die Ölphase ferner einen Gehalt an cyclischen oder linearan Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wässrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z. B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

Erfindungsgemäße Emulsionen sind vorteilhaft und enthalten z. B. die genannten Fette, Öle, Wachse und anderen Fettkörper, sowie Wasser und einen Emulgator, wie er üblicherweise für einen solchen Typ der Formuierung verwendet wird.

In einer bevorzugten Ausführungsform enthalten die erfindungsgemäßen Zubereitungen hydrophile Tenside.

Die hydrophilen Tenside werden bevorzugt gewählt aus der Gruppe der Alkylglucoside, der Acyllactylate, der Betaine sowie der Cocoamphoacetate.

Die Alkylglucoside werden ihrerseits vorteilhaft gewählt aus der Gruppe der Alkylglucoside, welche sich durch die Strukturformel

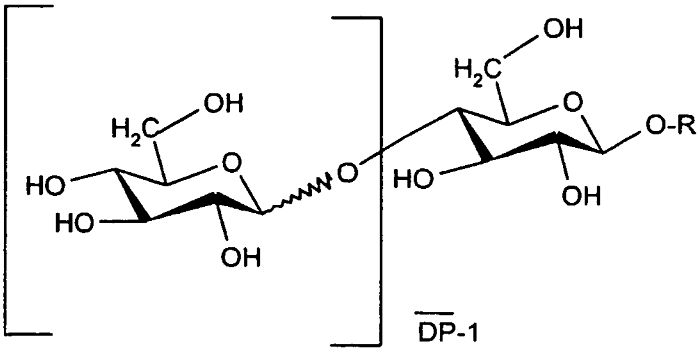

auszeichnen, wobei R einen verzweigten oder unverzweigten Alkylrest mit 4 bis 24 Kohlenstoffatomen darstellt und wobei $\overline{DP}$ einen mittleren Glucosylierungsgrad von bis zu 2 bedeutet. Der Wert $\overline{DP}$ repräsentiert den Glucosidierungsgrad der erfindungsgemäß verwendeten Alkylglucoside und ist definiert als

$$\overline{DP} = \frac{p_1}{100} \cdot 1 + \frac{p_2}{100} \cdot 2 + \frac{p_3}{100} \cdot 3 + \ldots = \sum \frac{p_i}{100} \cdot i$$

Dabei stellen $p_1$, $p_2$, $p_3$ ... bzw. $p_i$ den Anteil der einfach, zweifach dreifach ... i-fach glucosylierten Produkte in Gewichtsprozenten dar. Erfindungsemäß vorteilhaft werden Produkte mit Glucosylierungsgraden von 1-2, insbesondere vorteilhaft von 1, 1 bis 1,5, ganz besonders vorteilhaft von 1,2-1,4, insbesondere von 1,3 gewählt.

Der Wert DP trägt den Umstande Rechnung, dass Alkylglucoside herstellungsedingt in der Regel Gemische aus Mono- und Oligoglucosiden darstellen. Erfindungsgemäß vorteilhaft ist ein relativ hoher Gehalt an Monoglucosiden, typischerweise in der Größenordnung von 40-70 Gew.-%.

Erfindungsgemäß besonders vorteilhaft verwendete Alkylglylcoside werden gewählt aus der Gruppe Octylglucopyranosid, Nonylglucopyranosid, Decylglucopyranosid, Undecylglucopyranosid, Dodecylglucopyranosid, Tetradecylglucopyranosid und Hexadecylglucopyranosid.

Es ist ebenfalls von Vorteil, natürliche oder synthetische Roh- und Hilfsstoffe bzw. Gemische einzusetzen, welche sich durch einen wirksamen Gehalt an den erfindungsgemäß verwendeten Wirkstoffen auszeichnen, beispielsweise Plantaren® 1200 (Henkel KGaA), Oramix® NS 10 (Seppic).

Die Acyllactylate werden ihrerseits vorteilhaft gewählt aus der Gruppe der Substanzen, welche sich durch die Strukturformel

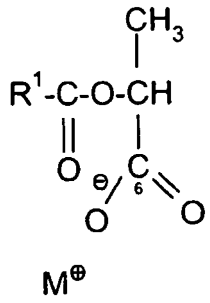

auszeichnen, wobei $R^1$ einen verzweigten oder unverzweigten Alkylrest mit 1 bis 30 Kohlenstoffatomen bedeutet und $M^+$ aus der Gruppe der Alkaliionen sowie der Gruppe der mit einer oder mehreren Alkyl- und/oder mit einer oder mehreren Hydroxyalkylresten substituierten Ammoniumionen gewählt wird bzw. dem halben Äquivalent eines Erdalkalions entspricht.

Vorteilhaft ist beispielsweise Natriumisostearyllactylat, beispielsweise das Produkt Pathionic® ISL von der Gesellschaft American Ingredients Company.

Die Betaine werden vorteilhaft gewählt aus der Gruppe der Substanzen, welche sich durch die Strukturformel

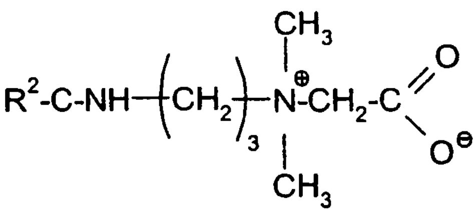

auszeichnen, wobei $R^2$ einen verzweigten oder unverzeigten Alkylrest mit 1 bis 30 Kohlenstoffatomen bedeutet.

Insbesondere vorteilhaft bedeutet $R^2$ einen verzweigten oder unverzweigten Alkylrest mit 6 bis 12 Kohlenstoffatomen.

Vorteilhaft ist beispielsweise Capramidopropylbetain, beispielsweise das Produkt Tego® Betain 810 von der Gesellschaft Th. Goldschmidt AG.

Als erfindungsgemäß vorteilhaftes Cocoamphoacetat wird beispielsweise Natriumcocoamphoacetat gewählt, wie es unter der Bezeichnung Miranol® Ultra C32 von der Gesellschaft Miranol Chemical Corp. erhältlich ist.

Die erfindungsgemäßen Zubereitungen sind vorteilhaft **dadurch gekennzeichnet, dass** das oder die hydrophilen Tenside in Konzentrationen von 0,01-20 Gew.-% bevorzugt 0,05-10 Gew.-%, besonders bevorzugt 0,1-5 Gew.-%, jeweils bezogen auf das Gesamtgewicht der Zusammensetzung, vorliegt oder vorliegen.

Zu Anwendung werden die erfindungsgemäßen kosmetischen und dermatologischen Zubereitungen in der für Kosmetika üblichen Weise aufdie Haut und/oder die Haare in ausreichender Menge aufgebracht.

Erfindungsgemäße kosmetische und dermatologische Zubereitungen können in verschiedenen Formen vorliegen. So können sie z. B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsion, beispielsweise vom Typ Waser-in-Öl-in-Wasser (W/O/W), ein Gel, einen festen Stift, eine Salbe oder auch ein Aerosol darstellen. Es ist auch vorteilhaft, Ectoine in verkapselter Form darzureichen, z. B. in Kollagenmatrices und anderen üblichen Verkapselungsmaterialien, z. B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt. Bevorzugt werden Emulsionen. O/W-Emulsinen werden besonders bevorzugt. Emulsionen, W/O-Emulsionen und O/W-Emulsionen sind in üblicher Weise erhältlich.

Als Emulgatoren können beispielsweise die bekannten W/O- und O/W-Emulgatoren verwendet werden. Es ist vorteilhaft, weitere übliche Co-emulgatoren in den erfindungsgemäßen bevorzugten O/W-Emulsionen zu verwenden.

Erfindungsgemäß vorteilhaft werden als Co-Emulgatoren beispielsweise O/W-Emulgatoren gewählt, vornehmlich aus der Gruppe der Substanzen mit HLB-Werten von 11-16, ganz besonders vorteilhaft mit HLB-Werten von

14,5-15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkhole, Cetylalkohole, Cetylstearylalkohole (Cetearylalkohole) zu wählen. Insbesondere bevorzugt sind: Polyethylenglycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethylenglycol(17)-stearylether (Steareth-17),Polyethylenglycol(18) stearylether (Steareth-18), Polyethylenglycol(19)stearylether (Steareth-19), Polyethylenglycol(20)-stearylether (Steareth-20), Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol(15)isostearylether (Isosteareth-15), Polyethylenglycol(16)-isostearylether (Isosteareth-16), Polyethylenglycol(17)isostearylether (Isosteareth-17), Polyethylenglycol(18)isostearylether (Isosteareth-18), Polyethylenglycol(19)isostearylether (Isosteareth-19), Polyethylenglycol(20)isostearylether (Isosteareth-20), Polyethylenglycol(13)cetylether (Ceteth-13), Polyethylenglycol(14)cetylether (Ceteth-14), Polyethylenglycol(15)cetylether (Ceteth-15), Polyethylenglycol(16)cetylether (Ceteth-16), Polyethylenglycol(17)cetylether (Ceteth-17), Polyethylenglycol(18)-cetylether (Ceteth-18), Polyethylenglycol(19)cetylether (Ceteth-19), Polyethylen-glycol(20)cetylether (Ceteth-20), Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(16)isocetylether (Isoceteth-16), Polyethylenglycol(17)-isocetylether (Isoceteth-17), Polyethylenglycol(18)isocetylether (Isoceteth-18), Polyethylenglycol (19)isocetylether (Isoceteth-19), Polyethylenglycol(20)isocetylether (Isoceteth-20), Polyethylenglycol(12)oleylether (Oleth-12), Polyethylenglycol(13)oleylether (Oleth-13), Polyethylenglycol(14)oleylether (Oleth-14), Polyethylenglycol (15)oleylether (Oleth-15), Polyethylenglycol(12)laurylether (Laureth-12), Polyethylenglycol(12)-isolaurylether (Isolaureth-12), Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(1 6)cetylstearylether (Ceteareth-16), Polyethylenglycol(1 7)-cetylstearylether (Ceteareth-17), Polyethylenglycol(18)cetylstearylether (Ceteareth-18), Polyethylenglycol(19)cetylstearylether (Ceteareth-19), Polyethylenglycol(20)cetylstearylether (Ceteareth-20).

Es ist ferner von Vorteil, die Fettsäureethoxylate ausfolgender Gruppe zu wählen:

Polyethylenglycol(20)stearat, Polyethylenglycol(21)stearat,
Polyethylenglycol(22)stearat, Polyethylenglycol(23)stearat,
Polyethylenglycol(24)stearat, Polyethylenglycol(25)stearat,
Polyethylenglycol(12)isostearat, Polyethylenglycol(13)isostearat,
Polyethylenglycol(14)isostearat, Polyethylenglycol(15)isostearat,
Polyethylenglycol(16)isostearat, Polyethylenglycol(17)isostearat,
Polyethylenglycol(18)isostearat, Polyethylenglycol(19)isostearat,
Polyethylenglycol(20)isostearat, Polyethylenglycol(21)isostearat,
Polyethylenglycol(22)isostearat, Polyethylenglycol(23)isostearat,
Polyethylenglycol(24)isostearat, Polyethylenglycol(25)isostearat,
Polyethylenglycol(12)oleat, Polyethylenglycol(13)oleat,
Polyethylenglycol(1 4)oleat, Polyethylenglycol(15)oleat,
Polyethylenglycol(16)oleat, Polyethylenglycol(17)oleat,
Polyethylenglycol(18)oleat, Polyethylenglycol(19)oleat,
Polyethylenglycol(20)oleat,

Als ethoxylierte Alkylethercarbonsäure bzw. deren Salz kann vorteilhaft das Natriumlaureth-11-carboxylat verwendet werden. Als Alkylethersulfat kann Natrium Laureth1-4sulfat vorteilhaft verwendet werden. Als ethoxyliertes Cholesterinderivat kann vorteilhaft Polyethylenglycol(30)Cholesterylether verwendet werden. Auch Polyethylenglycol(25) Sojasterol hat sich bewährt. Als ethoxylierte Triglyceride können vorteilhaft die Polyethylenglycol(60) Evening Primrose Glycerides verwendet werden (Evening Primrose = Nachtkerze). Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/caprinat, Polyethylenglycol-(20)glyceryloleat, Polyethylenglycol(20)glycerylisostearat, Polyethylenglycol(18)glyceryloleat/cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als fakultative, dennoch erfindungsgemäß gegebenenfalls vorteilhafte W/O-Emulgatoren können eingesetzt werden:

Fettalkohole mit 8 bis 30 Kohlenstoffatomen, Monoglycerinester gesättigter und/oder ungesättigter, verzweigter

und/oder unverzweigter Alkancarbonsäuren einer Kettenlänge von 8 bis 24, insbesondere 12-18 C-Atome, Diglycerinester gesättigter und/oder ungesättigter, verzweigter und/oder unverzweigter Alkancarbonsäuren einer Kettenlänge von 8 bis 24, insbesondere 12-18 C-Atomen, Monoglycerinether gesättigter und/oder ungesättigter, verzweigter und/oder unverzweigter Alkohole einer Kettenlänge von 8 bis 24, insbesondere 12-18 C-Atomen, Diglycerinether gesättigter und/oder ungesättigter, verzweigter und/oder unverzweigter Alkhole einer Kettenlänge von 8 bis 24, insbesondere 12-18 C-Atomen, Propylenglycolester gesättigter und/oder ungesättigter, verzweigter und/oder unverzweigter Alkancarbonsäuren einer Kettenlänge von 8 bis 24, insbesondere 12-18 C-Atomen sowie Sorbitanester gesättigter und/oder ungesättigter, verzweigter und/oder unverzweigter Alkancarbonsäuren einer Kettenlänge von 8 bis 24, insbesondere 12-18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat oder PEG-30-dipolyhydroxystearat.

Erfindungsgemäß bevorzugte Zubereitungen eignen sich besonders zum Schutz menschlicher Haut gegen Alterungsprozesse sowie vor oxidativem Stress, d.h. gegen Schädigungen durch Radikale, wie sie z.B. durch Sonneneinstrahlung, Wärme oder andere Einflüsse erzeugt werden. Dabei liegt sie in verschiedenen, für diese Anwendung üblicherweise verwendeten Darreichungsformen vor. So kann sie insbesondere als Lotion oder Emulsion, wie als Creme oder Milch (O/W, W/O, O/W/O, W/O/W), in Form ölig-alkoholischer, ölig-wässriger oder wässrigalkoholischer Gele bzw. Lösungen, als feste Stifte vorliegen oder als Aerosol konfektioniert sein.

Die Zubereitung kann kosmetische Adjuvantien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfums, Wachse, Lanolin, Treibmittel, Farbstoffe und/oder Pigmente, welche das Mittel selbst oder die Haut färben, und andere in der Kosmetik gewöhnlich verwendete Ingredienzien.

Als Farbstoffe werden bevorzugt zugelassene Farbstoffe verwendet, die in der Kosmetikverordnung, Anlage 3 als Positivliste aufgeführt sind.

Als Konservierungsstoffe werden bevorzugt zugelassene Konservierungsstoffe verwendet, die in der Kosmetikverordnung, Anlage 6 als Positivliste aufgeführt sind oder auch antimikrobiellen Pigmente, wie beispielsweise in WO 2004/0092283 oder WO 2004/091567 beschrieben.

Geeignete Konservierungsstoffe sind daher auch Alkylester der p-Hydroxybenzoesäure, Hydantoinderivate, Propionat-Salze oder eine Vielzahl von Ammoniumverbindungen.

Ganz besonders bevorzugte Konservierungsstoffe sind Methylparaben, Propylparaben, Imidazolidinyl-Harnstoff, Natrium-dehydroxyacetat oder Benzylalkohol. Konservierungsmittel werden in Mengen zwischen 0.5 bis 2 Gew.-% eingesetzt.

Emollients oder Weichmacher werden oft in kosmetische Zubereitungen eingearbeitet. Sie werden bevorzugt in 0.5 bis 50 Gew.-%, bevorzugt zwischen 5 und 30 Gew.-% bezogen auf die Gesamtzusammensetzung eingesetzt. Generell können Weichmacher in Klassen eingeordnet werden, wie beispielsweise die Kategorie der Ester, Fettsäuren oder Fettalkohole, Polyole, Kohlenwasserstoffe und Öle enthaltend mindestens eine Amidstruktur-Einheit.

Repräsentative Öle enthaltend mindestens eine Amidstruktur-Einheit zusammen mit ihrer Synthese sind insbesondere in EP 1044676 und EP 0928608 beschrieben. Eine besonders bevorzugt angegebene Verbindung ist Isopropyl-N-lauroylsarcosinat, welches unter der Produktbezeichnung Eldew SL-205 von Ajinomoto kommerziell erhältlich ist.

Unter den Estern können Mono- oder Diester ausgewählt warden. Beispiele sind diesbezüglich Dibutyl-adipat, Diethyl-sebacat, Disopropyldimerat oder Dioctyl-succinat. Verzweigte Fettsäureester sind beispielsweise 2-Ethyl-hexylmyristat, Isopropyl-stearat oder Isostearylpalmitat. Tribasische Ester sind beispielsweise Trisopropyl-trilinoleat oder Trilauryl-citrat. Geradkettige Fettsäureester sind beispielsweise Laurylpalmitat, Myristyl-lactat, Oleyl-eurcat oder Stearyloleat. Bevorzugte Ester sind Coco-Caprylate/Caprate (= INCI-Bezeichnung, es sind Ester aus Kokosfettalkoholen mit gesättigten mitelkettigen Fettsäuren), Propylenglycol-myristyl-ether-acetat, Diisopropyl-adipat oder Cetyloctanoat.

Geeignete Fettalkohole und -säuren sind Verbindungen, die 10 bis 20 C-Atome haben. Besonders bevorzugte Verbindungen sind Cetyl-, Myristyl-, Palmitin- oder Stearinalkohol oder -säure.

Als Polyole eignen sich lineare oder verzweigtkettige Alkylpolyhydroxyverbindungen, beispielsweise Propylenglycol, Sorbitol oder Glycerin. Einsetzbar sind jedoch auch polymere Polyole, beispielsweise Polypropylenglycol oder Polyethylenglycol. Butylen- und Propylenglycol sind auch besonders geeignete Verbindungen zur Verstärkung des Eindringungsvermögens.

Beispielhafte Kohlenwasserstoffe als Weichmacher sind Verbindungen, die generell 12 bis 30 C-Atome haben.

Spezielle Beispiele sind Arylalkylbenzoate, Alkylbenzoate, Mineralöle, Vaseline, Squalene oder Isoparaffine.

Weitere Emollients oder Hydrophobiermittel sind bevorzugt $C_{12}$ bis $C_{15}$ Alkylbenzoate, Dioctyladipat, Octylstearat, Octyldodecanol, Hexyllaurat, Octyldodecyl-neopentanoat, Cyclomethicone, Dicapryl-ether, Dimethicone, Phenyl-trimethicone, Isopropyl-myristat, Capriylic/Capric-glyceride, Propylenglycol-dicaprylat/dicaprat oder Decyl-oleat.

Eine weitere Kategorie funktioneller Inhaltsstoffe von kosmetischen Zubereitungen im Sinne der Erfindung sind Verdickungsmittel. Verdickungsmittel werden in der Regel in Mengen zwischen 0.1 bis 20 Gew.-%, vorzugsweise zwischen 0.5 bis 10 Gew.-% bezogen auf die Gesamtmenge eingesetzt. Beispielhaft für diese Verbindungen sind vernetzte Polyacrylat-Materialien, kommeziell erhältlich under der Marke Carbopol von B. F. Goodrich Company. Verwendet werden können auch Verdickungsmittel, wie Xanthan-Gum, Carrageenan-Gum, Gelatin-Gum, Karayagummi, Pectin-Gum oder Johannisbrotkernmehl.

Unter gewissen Umständen ist es möglich, dass eine Verbindung sowohl ein Verdickungsmittel als auch ein Weichmacher sein kann. Beispiele hierfür sind Silicon-Gums (kinematische Viskosität > 10 Centistokes), Ester wie beispielsweise Glycerolstearat oder Cellulosederivate, beispielsweise Hydroxypropylcellulose.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglykol, Glycerin und Sorbit.

Eine bevorzugte Ausführungsform der Erfindung ist eine Emulsion, welche als Schutzcreme oder -milch vorliegt und außer der oder den Verbindungen der Formel I beispielsweise Fettalkohole, Fettsäuren, Fettsäureester, insbesondere Triglyceride von Fettsäuren, Lanolin, natürliche und synthetische Öle oder Wachse und Emulgatoren in Anwesenheit von Wasser enthält.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder öligalkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Die erfindungsgemäße Zubereitung kann auch als alkoholisches Gel vorliegen, welches einen oder mehrere Niedrigalkohole oder -polyole, wie Ethanol, Propylenglykol oder Glycerin, und ein Verdickungsmittel, wie Kieselerde umfaßt. Die ölig-alkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpem.

Ist eine Zubereitung als Aerosol konfektioniert, verwendet man in der Regel die üblichen Treibmittel, wie Alkane, Fluoralkane und Chlorfluoralkane.

Die kosmetische Zubereitung kann auch zum Schutz der Haare gegen fotochemische Schäden verwendet werden, um Veränderungen von Farbnuancen, ein Entfärben oder Schäden mechanischer Art zu verhindern. In diesem Fall erfolgt geeignet eine Konfektionierung als Shampoo, Lotion, Gel oder Emulsion zum Ausspülen, wobei die jeweilige Zubereitung vor oder nach dem Shampoonieren, vor oder nach dem Färben oder Entfärben bzw. vor oder nach der Dauerwelle aufgetragen wird. Es kann auch eine Zubereitung als Lotion oder Gel zum Frisieren und Behandeln, als Lotion oder Gel zum Bürsten oder Legen einer Wasserwelle, als Haarlack, Dauerwellenmittel, Färbe- oder Entfärbemittel der Haare gewählt werden. Die Zubereitung kann außer der oder den Verbindungen der Formel I verschiedene, in diesem Mitteltyp verwendete Adjuvantien enthalten, wie Grenzflächen aktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien.

Die Zubereitungen, wie zuvor beschrieben, können die genannten notwendigen oder optionalen Bestandteile umfassen oder enthalten, daraus im wesentlichen oder daraus bestehen. Alle Verbindungen oder Komponenten, die in den Zubereitungen verwendet werden können, sind entweder bekannt und käuflich erwerbbar oder können nach bekannten Verfahren synthetisiert werden.

Weitere Gegenstände der vorliegenden Erfindung sind ein Verfahren zur Herstellung einer Zubereitung, welches dadurch gekennzeichnet ist, dass mindestens eine Verbindung der Formel I mit Resten wie oben beschrieben mit einem kosmetisch, pharmazeutisch oder dermatologisch oder für Nahrungsmittel geeigneten Träger vermischt wird, und die Verwendung einer Verbindung der Formel I zur Herstellung einer Zubereitung, insbesondere mit antioxidanten Eigenschaften.

Die erfindungsgemäßen Zubereitungen können dabei mit Hilfe von Techniken hergestellt werden, die dem Fachmann wohl bekannt sind.

Das Vermischen kann ein Lösen, Emulgieren oder Dispergieren der Verbindung gemäß Formel I in dem Träger zur Folge haben.

Gegenstand der Erfindung ist auch ein Verfahren zur Herstellung der Verbindungen der Formel I, wobei $R^6$ unabhängig voneinander Alkyl mit 1 bis 18 C-Atomen, bevorzugt Alkyl mit 1 bis 5 C-Atomen, besonders bevorzugt Methyl, und $R^5$ = OH bedeutet, **dadurch gekennzeichnet, dass** man eine Verbindung der Formel II

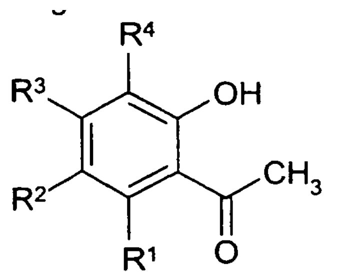

II

,

wobei $R^1$ bis $R^4$ eine der zuvor genannten Bedeutungen haben können, mit einem Reagenz ausgewählt aus $POCl_3$, Phosgen oder Trifluorsulfonsäureanhydrid und einem Arylalkyl-, Diaryl- oder Dialkylformamid zu einem Zwischenprodukt der Formel III

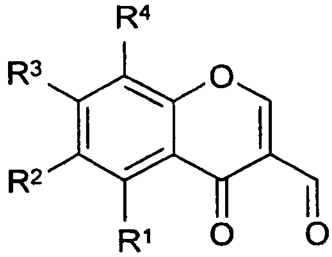

III

,

wobei $R^1$ bis $R^4$ eine der zuvor genannten Bedeutungen haben können, umsetzt
und dieses anschließend mit
einem Phosphorigsäuredialkylester umsetzt.

Die Alkylgruppen des Phosphorigsäuredialkylesters bilden $R^6$.
Die Umsetzung zu den Zwischenprodukten der Formel III, wie zuvor beschrieben, wird auch Vilsmeier-Reaktion genannt.

Beispiele für Arylalkyl-, Diaryl- oder Dialkylformamide sind N-Phenyl-N-methylformamid, N,N-Diphenylformamid, N,N-Dimethylformamid, N,N-Diethylformamid, N,N-Dibutylformamid. Anstelle eines Formamids kann auch N,N-Formylpiperidin eingesetzt werden.

Bevorzugt findet die Vilsmeier-Reaktion in Gegenwart von $POCl_3$ und Dimethylformamid statt.
Die Vilsmeier-Reaktion erfolgt vorzugsweise unter Inertgasbedingungen. Vorteilhaft wird $POCl_3$ oder ein anderes Reagenz aus der angegebenen Gruppe bei Temperaturen <5°C eingebracht. Die eigentliche Reaktionstemperatur liegt zwischen -50 und 75°C, bevorzugt zwischen -20 und 30°C. Besonders bevorzugt erfolgt die Umsetzung bei Raumtemperatur.

Die Umsetzung der Zwischenprodukte der Formel III mit Phosphorigsäuredialkylester findet vorzugsweise unter Inertgasbedingungen und bei Reaktionstemperaturen zwischen 20 und 100°C, bevorzugt zwischen 25 und 85°C statt. Als Katalysator kann beispielsweise ein Trialkylphosphit, CsF, KF, DBU (Diaza-bicyclo-undec-7-en), Natrium, Natriummethoxid, Triethylamin oder auch NaOH eingesetzt werden, wobei jedoch bei der Verwendung von NaOH noch ein weiterere Phasen-Transfer-Katalysator benötigt wird. Bevorzugt wird ein Trialkylphosphit eingesetzt.
Vorzugsweise sind die Alkylgruppen des Trialkylphosphits identisch zu den Alkylgruppen des Phosphorigsäuredialkylesters und stehen für $R^6$ in den Verbindungen der Formel I mit den zugehörigen offenbarten Bedeutungen.

Durch Esterhydrolyse, beispielsweise durch Umsetzung mit Salzsäure, erhält man Verbindungen der Formel I, in denen $R^6$ jeweils H bedeutet.

In einem anschließenden Reduktionsschritt, beispielsweise durch Reduktion mit Iodwasserstoffsäure in Gegenwart von rotem Phosphor, können Verbindungen der Formel I erhalten werden, in denen $R^5$ H bedeutet.
Die Reduktion erfolgt beispielsweise in Essigsäure bei Temperaturen zwischen 10 und 150°C, bevorzugt zwischen 20 und 125°C, besonders bevorzugt bei 100 bis 120°C.

Die Umwandlung in Salze der Phosphonsäuren der Formel I gelingt beispielsweise durch Zusatz eines Alkali- oder Erdalkalimetall-hydroxids, - carbonats oder -bicarbonats in einem polaren Lösungsmitel, beispielsweise in Ethanol, Methanol oder Isopropanol.

Die angegebenen Verbindungen der Formel II sowie die weiteren Reaktanden in der Synthese sind kommerziell erhältlich oder durch Synthesen zugänglich, die dem Fachmann aus der Literatur wohl bekannt sind. Die Auswahl der geeigneten Reaktionsbedingungen gehört zum Standard des Synthesefachmanns.

Es wurde auch festgestellt, dass Verbindungen der Formel I stabilisierend auf Zubereitungen wirken können.

Bei der Verwendung in entsprechenden Produkten bleiben diese daher auch länger stabil und verändern ihr Aussehen nicht. Insbesondere bleibt auch bei längerdauernder Anwendung bzw. längerer Lagerung die Wirksamkeit der Inhaltsstoffe, z.B. Vitamine, erhalten. Dies ist unter anderem besonders vorteilhaft bei Zusammensetzungen zum Schutz der Haut gegen die Einwirkung von UV-Strahlen, da diese Kosmetika besonders hohen Belastungen durch die UV-Strahlung ausgesetzt sind.

Die positiven Wirkungen von Verbindungen der Formel I ergeben deren besondere Eignung zur Verwendung in kosmetischen oder pharmazeutischen Zubereitungen.

Ebenso positiv sind die Eigenschaften von Verbindungen mit der Formel I zu werten für eine Verwendung in Nahrungsmitteln oder als Nahrungsergänzungsmittel oder als "functional food". Die weiteren zu Nahrungsmitteln ausgeführten Erläuterungen gelten sinngemäß auch für Nahrungsergänzungsmittel und für "functional food".

Die Nahrungsmittel, die nach der vorliegenden Erfindung mit einer oder mehreren Verbindungen der Formel I angereichert werden können, umfassen alle Materialien, die für den Verzehr durch Tiere oder für den Verzehr durch Menschen geeignet sind, beispielsweise Vitamine und Provitamine davon, Fette, Mineralien oder Aminosäuren ". (Die Nahrungsmittel können fest sein aber auch flüssig, also als Getränk vorliegen). Weitere Gegenstände der vorliegenden Erfindung sind dementsprechend Zubereitungen, die Nahrungsmittel oder Nahrungsergänzungsmittel sind und entsprechende Träger enthalten.

Nahrungsmittel, die nach der vorliegenden Erfindung mit einer oder mehreren Verbindungen der Formel I angereichert werden können, sind beispielsweise auch Nahrungsmittel, die aus einer einzigen natürlichen Quelle stammen, wie z.B. Zucker, ungesüßter Saft, Nektar oder Püree von einer einzigen Pflanzenspezies, wie z.B. ungesüßter Apfelsaft (z.B. auch eine Mischung verschiedener Sorten Apfelsaft), Grapefruitsaft, Orangensaft, Apfelkompott, Aprikosennektar, Tomatensaft, Tomatensoße, Tomatenpüree usw. Weitere Beispiele für Nahrungsmittel sind Korn oder Getreide einer einzigen Pflanzenspezies und Materialien, die aus derartigen Pflanzenspezies hergestellt werden, wie z.B. Getreidesirup, Roggenmehl, Weizenmehl oder Haferkleie. Auch Mischungen von derartigen Nahrungsmitteln sind geeignet, um nach der vorliegenden Erfindung mit einer oder mehreren Verbindungen der Formel I angereichert zu werden, beispielsweise Multivitaminpräparate, Mineralstoffmischungen oder gezuckerter Saft. Als weitere Beispiele für Nahrungsmittel seien Nahrungsmittelzubereitungen, beispielsweise zubereitete Cerealien, Gebäck, Mischgetränke, speziell für Kinder zubereitete Nahrungsmittel, wie Joghurt, Diätnahrungsmittel, kalorienarme Nahrungsmittel oder Tierfutter, genannt.

Die Nahrungsmittel, die nach der vorliegenden Erfindung mit einer oder mehreren Verbindungen der Formel I angereichert werden können, umfassen somit alle genießbaren Kombinationen von Kohlehydraten, Lipiden, Proteinen, anorganischen Elementen, Spurenelementen, Vitaminen, Wasser oder aktiven Metaboliten von Pflanzen und Tieren.

Die Nahrungsmittel, die nach der vorliegenden Erfindung mit einer oder mehreren Verbindungen der Formel I angereichert werden können, werden vorzugsweise oral angewendet, z.B. in Form von Speisen, Pillen, Tabletten, Kapseln, Pulver, Sirup, Lösungen oder Suspensionen.

Die mit einer oder mehreren Verbindungen der Formel I angereicherten erfindungsgemäßen Nahrungsmittel können mit Hilfe von Techniken hergestellt werden, die dem Fachmann wohl bekannt sind.

Durch ihre Wirkung als Antioxidationsmittel bzw. als Radikalfänger eignen sich Verbindungen der Formel I auch als Arzneimittelinhaltsstoff. Sie wirken dabei unterstützend oder substituierend zu natürlichen Mechanismen, welche Radikale im Körper abfangen. Die Verbindungen der Formel I können in ihrer Wirkung teilweise mit Radikalfängern wie Vitamin C verglichen werden. Verbindungen der Formel I können beispielsweise zur vorbeugenden Behandlungen von Entzündungen und Allergien der Haut sowie in bestimmten Fällen zur Verhütung bestimmter Krebsarten verwendet werden. Insbesondere eignen sich Verbindungen der Formel I zur Herstellung eines Arzneimittels zur Behandlung von Entzündungen, Allergien und Irritationen, insbesondere der Haut. Ferner können Arzneimittel hergestellt werden in einer Wirkung als Venentonikum, als Mittel zur Erhöhung der Festigkeit von Blutkapillaren, als Hemmstoff für Cuperose, als Hemmstoff chemischer, physikalischer oder aktinischer Erytheme, als Mittel zur Behandlung empfindlicher Haut, als Dekongestionsmittel, als Entwässerungsmittel, als Mittel zum Schlankmachen, als Antifaltenmittel, als Stimulatoren der Synthese von Komponenten der extrazellulären Matrix, als stärkendes Mittel zur Verbesserung der Hautelastizität und als Antialterungsmittel. Weiter zeigen in diesem Zusammenhang bevorzugte Verbindungen der Formel I antiallergische und antiinflammatorische und antiirritative Wirkungen. Sie eignen sich daher zur Herstellung von Arzneimitteln zur Behandlung von Entzündungen oder allergischen Reaktionen.

Im folgenden wird die Erfindung anhand von Beispielen näher erläutert. die Erfindung ist im gesamten beanspruchten Bereich ausführbar und nicht auf die hier genannten Beispiele beschränkt.

**Beispiele**

**Beispiel 1 Herstellung von [(7,8-Dihydroxy-4-oxo-4H-chromen-3-yl)-hydroxymethyl]-phosphonsäure**

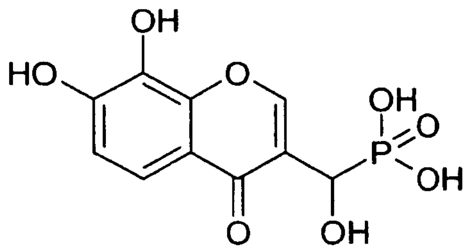

Durchführung:

**1.Stufe:**

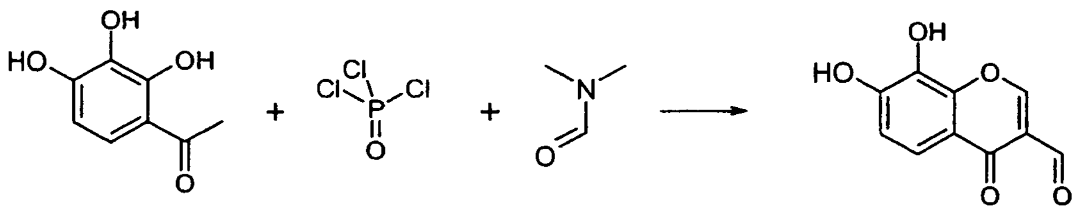

10 g (59,5 mmol) 2,3,4-Trihydroxyacetophenon werden in 120 mL N,N-Dimethylformamid gelöst, bei -50°C (Trockeneis/Ethanol) vorgelegt und anschließend 21,8 ml (237,9 mmol) Phosphorylchlorid langsam in ca. 30min zugetropft. Anschließend wird die klare Lösung 30 min. bei -20°C gerührt, das Kältebad entfernt und über Nacht bei Raumtemperatur nachgerührt.

Die komplette Reaktionslösung wird auf ca. 200ml Eis/Wasser gegossen und die entstehende Suspension wird abgesaugt und mit Wasser nachgewaschen. Der erhaltene Feststoff wird über Nacht im Vakuumtrockenschrank bei 45°C und 200mbar getrocknet.

$^{1}H$ NMR (500MHz) in DMSO δ (ppm):.7.0 (d, 1H), 7.5 (d, 1H), 8.8 (s, 1H), 10.1 (s, 1H), 10.6 (bs, OH).

$^{13}C$ NMR (75MHz) in DMSO δ (ppm): 114.8, 115.5, 117.7, 119, 133.6, 146.3, 151.3, 162.3, 174.5, 188.6.

ESI-MS (m/z): 206.

**2.Stufe:**

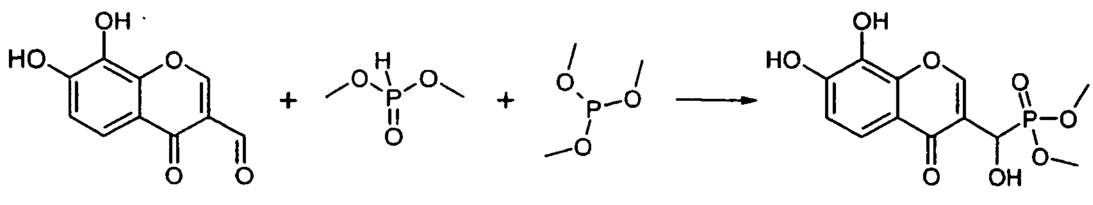

4,3 g (20,858 mmol) 7,8-Dihydroxy-4-oxo-4H-1-benzopyran-3-carbaldehyd werden bei RT (RT = Raumtemperatur) unter Argon in 19 mL Phosphorigsäuredimethylester suspendiert und dann ein paar Tropfen Trimethylphosphit zugegeben. Die Suspension wird daraufhin 1,5 h bei 85°C gerührt und über Nacht bei RT nachgerührt.

Zur Aufarbeitung wird die Reaktionslösung auf 200 mL Dichlormethan gegeben und 1 h bei RT gerührt. Der Phosphonsäureester fällt als Feststoff aus.

$^{1}H$ NMR (250MHz) in DMSO δ (ppm):.3.6 (d, 3H), 3.7 (d, 3H), 5.25 (bd, 1H), 6.2 (bs, OH), 6.95 (d, 1H), 7.45 (d, 1H), 8.25 (d, 1H), 9.45 (bs, OH), 10.53 (bs, OH).

$^{13}C$ NMR (63MHz) in DMSO δ (ppm): 53(d), 53.4(d), 54.8, 58.1, 60.8, 114.4, 115.4, 116.5, 120.6, 133.0, 146.6, 150.3, 155.2, 174.1.

$^{31}$P NMR (100MHz) in DMSO δ (ppm): 25.3.

## 3. Stufe:

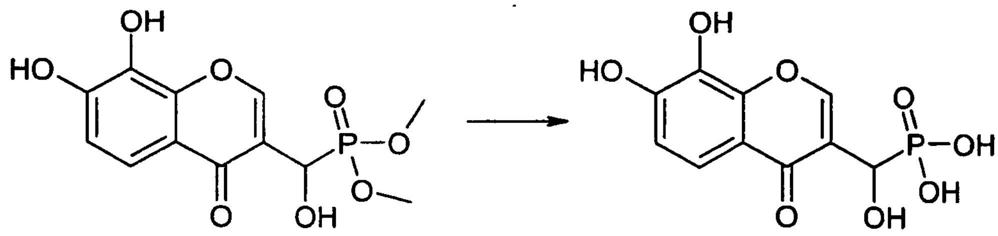

5,2 g (16,44 mmol) [(7,8-Dihydroxy-4-oxo-4*H*-1-benzopyran-3-yl)-hydroxymethyl]-phosphonsäuredimethylester werden in 40 mL 2N Salzsäure suspendiert und zum Siedepunkt erwärmt. Nach vollständiger Hydrolyse wird die Reaktionslösung auf RT abgekühlt, wobei die Phosphonsäure als Feststoff ausfällt. Nach Abfiltrieren wird mit wenig Wasser gewaschen und getrocknet.
$^{1}$H NMR (250MHz) in DMSO δ (ppm):.5.0 (d, 1H), 6.9 (d, 1H), 7.4 (d, 1H), 8.2 (d, 1H), 9.4 (bs, OH), 10.25 (bs, OH).
$^{13}$C NMR (63MHz) in DMSO δ (ppm): 59.1, 61.8, 114.2, 115.4, 116.8, 121.9, 132.9, 146.5, 150.0, 154.7.
$^{31}$P NMR (100MHz) in DMSO δ (ppm) : 18.8.
ESI-MS (m/z): 288.

**Beispiel 2:**

Analog zu Beispiel 1 werden

a) 2,4-Dihydroxy-3-methyl-acetophenon in Dimethylformamid mit Phosphorylchlorid und anschließend mit Phosphorigsäuredimethylester umgesetzt. Man erhält [(7-Hydroxy-8-methyl-4-oxo-4H-chromen-3-yl)hydroxymethyl]-phosphonsäuredimethylester.
$^{1}$H NMR (300MHz) in DMSO δ (ppm): 2.25 (s, 3H), 3.66 (d, 3H), 3.74 (d, 3H), 5.27 (d, 1H), 7.03 (d, 1H), 7.81 (d, 1H), 8.3 (d, 1H).
Nach Esterhydrolyse erhält man [(7-Hydroxy-8-methyl-4-oxo-4H-chromen-3-yl)hydroxymethyl]-phosphonsäure.
$^{1}$H NMR (300MHz) in DMSO δ (ppm): 2.21 (s, 3H), 5.0 (d, 1H), 6.98 (d, 1H), 7.76 (d, 1H), 8.23 (d, 1H).

b) 2,4-Dihydroxy-acetophenon in Dimethylformamid mit Phosphorylchlorid und anschließend mit Phosphorigsäuredimethylester umgesetzt. Man erhält [(7-Hydroxy-4-oxo-4H-chromen-3-yl)hydroxymethyl]-phosphonsäuredimethylester.
$^{1}$H NMR (500MHz) in DMSO δ (ppm): 3.63 (d, 3H), 3.72 (d, 3H), 5.24 (d, 1H), 6.87 (d, 1H), 6.95 (dd, 1H), 7.93 (d, 1H), 8.21 (d, 1H).
Nach Esterhydrolyse erhält man [(7-Hydroxy-4-oxo-4H-chromen-3-yl)hydroxymethyl]-phosphonsäure.
$^{1}$H NMR (300MHz) in DMSO δ (ppm): 5.18 (d, 1H), 6.9 (d, 1H), 6.97 (dd, 1H), 7.97 (d, 1H), 8.21 (d, 1H).

c) 2,4-Dihydroxy-acetophenon in Dimethylformamid mit Phosphorylchlorid und anschließend mit Phosphorigsäurediethylester umgesetzt. Man erhält [(7-Hydroxy-4-oxo-4H-chromen-3-yl)hydroxymethyl]-phosphonsäurediethylester.

d) 2,5-Dihydroxy-acetophenon in Dimethylformamid mit Phosphorylchlorid und anschließend mit Phosphorigsäuredimethylester umgesetzt. Man erhält [(6-Hydroxy-4-oxo-4H-chromen-3-yl)hydroxymethyl]-phosphonsäuredimethylester.
$^{1}$H NMR (300MHz) in DMSO δ (ppm):.3.62 (d, 3H), 3.71 (d, 3H), 5.75 (d, 1H), 7.23 (dd, 1H), 7.35 (d, 1H), 7.49 (d, 1H), 8.27 (d, 1H).
Nach Esterhydrolyse erhält man [(6-Hydroxy-4-oxo-4H-chromen-3-yl)hydroxymethyl]-phosphonsäure.
$^{1}$H NMR (300MHz) in DMSO δ (ppm): 5.2 (d, 1H), 7.22 (dd, 1H), 7.33 (d, 1H), 7.51 (d, 1H), 8.24 (d, 1H).

e) 2-Hydroxy-5-methoxyacetophenon in Dimethylformamid mit Phosphorylchlorid und anschließend mit Phosphorigsäuredimethylester umgesetzt. Man erhält [(6-Methoxy-4-oxo-4H-chromen-3-yl)hydroxymethyl]-phosphonsäuredimethylester.

$^1H$ NMR (300MHz) in DMSO δ (ppm): 3.62 (d, 3H), 3.71 (d, 3H), 3.83 (s,3H), 5.28 (d, 1H), 7.35 (dd, 1H), 7.44 (d, 1H), 7.54 (d, 1H), 8.29 (d, 1H).
Nach Esterhydrolyse erhält man [(6-Methoxy-4-oxo-4H-chromen-3-yl)hydroxymethyl]-phosphonsäure.
$^1H$ NMR (300MHz) in DMSO δ (ppm): 3.84 (s,3H), 5.1 (d, 1H), 7.36 (dd, 1H), 7.44 (d, 1H), 7.56 (d, 1H), 8.26 (d, 1H).

f) 2,5-Dihydroxy-acetophenon in Dimethylformamid mit Phosphorylchlorid und anschließend mit Phosphorigsäuredibutylester umgesetzt. Man erhält [(6-Hydroxy-4-oxo-4H-chromen-3-yl)hydroxymethyl]-phosphonsäuredibutylester.

g) 2,3,4-Trihydroxy-acetophenon in Dimethylformamid mit Phosphorylchlorid und anschließend mit Phosphorigsäuredidodecylester umgesetzt. Man erhält [(7,8-Dihydroxy-4-oxo-4H-chromen-3-yl)hydroxymethyl]-phosphonsäuredidodecylester.
Nach Esterhydrolyse erhält man [(7,8-Dihydroxy-4-oxo-4H-chromen-3-yl)hydroxymethyl]-phosphonsäure.

h) 2,3,4-Trihydroxy-acetophenon in Dimethylformamid mit Phosphorylchlorid und anschließend mit Phosphorigsäuredioctadecylester umgesetzt. Man erhält [(7,8-Dihydroxy-4-oxo-4H-chromen-3-yl)hydroxymethyl]-phosphonsäuredioctadecylester.

**Beispiel 3:**

[(6-Hydroxy-4-oxo-4H-chromen-3-yl)-hydroxymethyl]-phosphonsäure, hergestellt nach Beispiel 2d) wird in Ethanol gelöst und anschließend eine 1 M-Lösung KOH so lange dazu gegeben, bis ein Feststoff ausfällt. Man erhält Kaliumsalze der [(6-Hydroxy-4-oxo-4H-chromen-3-yl)-hydroxymethyl]-phosphonsäure.

**Beispiel 4: Herstellung von [(6-Hydroxy-4-oxo-4H-chromen-3-yl)-methyl]-phosphonsäure**

[(6-Hydroxy-4-oxo-4H-chromen-3-yl)-hydroxymethyl]-phosphonsäure (10mmol), hergestellt nach Beispiel 2d), Iodwasserstoffsaeure (57%) (38mmol) und roter Phosphor (20mmol) werden zu Essigsäure (100mL) zugegeben. Diese Mischung wird bis zur vollständigen Umsetzung auf 110-115°C erhitzt. Anschließend wird die heiße Reaktionsmischung filtriert und der Rückstand mit zweimal 10 ml heißer Essigsäure gewaschen. Das Filtrat wird durch Zugabe von wässriger $Na_2SO_3$-Lösung entfärbt. Bei Temperaturen von 6 bis 10°C fällt ein Feststoff aus. Nach Filtration wird der Feststoff mit wenig kaltem Wasser gewaschen und getrocknet. Man erhält [(6-Hydroxy-4-oxo-4H-chromen-3-yl)-methyl]-phosphonsäure.
$^1H$ NMR (300MHz) in DMSO δ (ppm):.2.9 (d, 2H), 7.25 (dd, 1H), 7.38 (d, 1H), 7.5 (d, 1H), 8.2 (d, 1H).
$^{31}P$ NMR (100MHz) in DMSO δ (ppm): 16.8.
MS (m/z) : 222 ($M^+$).

**Beispiel 5: Antioxidative Eigenschaften für [(7,8-Dihydroxy-4-oxo-4H-chromen-3-yl)hydroxymethyl]-phosphonsäure, hergestellt nach Beispiel 1.**

a) Basis für die Bestimmung der antioxidativen Wirksamkeit ist der sog. DPPH-Test wie bei Bünger et. al. beschrieben [Buenger, J., Ackermann, H., Jentzsch, A., Mehling, A., Pfizner, I., Reiffen, K.-A., Schroeder, K.-R., and Wollenweber U., An interlaboratory comparison of methods used to assess antioxidant potentials, Int. J. Cosm. Sci., 28 (2006) 1-12]. Die antioxidative Wirksamkeit von [(7,8-Dihydroxy-4-oxo-4H-chromen-3-yl)hydroxymethyl]-phosphonsäure wird im DPPH Test ermittelt. Der $EC_{50}$-Wert liegt bei 0,24 μmol/L und spiegelt dabei hervorragenden Radikalfängereigenschaften wieder.

b) Basis für die Bestimmungen der antioxidativen Wirksamkeit ist auch das sogenannte TEAC Assay (Trolox Equivalent Antioxidant Activity Assay) wie bei Buenger et al. beschrieben [Buenger, J., Ackermann, H., Jentzsch, A., Mehling, A., Pfizner, I., Reiffen, K.-A., Schroeder, K.-R. und Wollenweber U., An interlaboratory comparison of methods used to assess antioxidant potentials, Int. J. Cosm. Sci., 28 (2006), 1-12].
ABTS [2,2'-azinobis-(3-ethyl-benzothiazolin-6-sulfonsäure)] reagiert mit Kalium Peroxodisulfat, indem ein stabiles Radikal-Kation erzeugt wird, welches bei 734 nm absorbiert. Das vermessene Antioxidans reduziert das Radikal-Kation und verursacht so eine Abschwächung des Absorptionsvermögens bei 734 nm. Die Messung der Absorption wird nach einer festen Zeitspanne von 6 Minuten durchgeführt. Das antioxidative Potential der vermessenen Substanz wird als Aktivität im Vergleich zu Trolox angegeben. Die vermessene Substanz kann während der Messung

in Wasser oder in Ethanol gelöst vorliegen.

Trolox

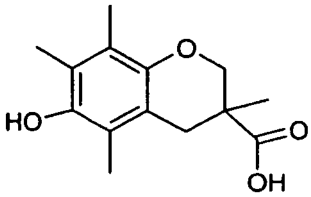

Der vermessene Wert liegt bei 0.93 im Vergleich zu 1.0 von Trolox, d.h. die erfindungsgemäße Verbindung zeigt ein hohes antioxidatives Potential.

**Beispiel 6: Zubereitungen**

Im folgenden werden beispielhaft Rezepturen für kosmetische Zubereitungen angegeben, die Verbindungen nach Beispielen 1 oder 2 enthalten. Im übrigen sind die INCI-Bezeichnungen der handelsüblichen Verbindungen angegeben.

UV-Pearl , OMC steht für die Zubereitung mit der INCI-Bezeichnung: Water (for EU: Aqua), Ethylhexyl Methoxycinnamate, Silica, PVP, Chlorphenesin, BHT; diese Zubereitung ist im handel unter der Bezeichnung Eusolex®UV Pearl™OMC von der Merck KGaA, Darmstadt erhältlich.
Die anderen in den Tabellen angegebenen UV-Pearl sind jeweils analog zusammengesetzt, wobei OMC gegen die angegebenen UV-Filter ausgetauscht wurde.

Tabelle 1 W/O-Emulsionen (Zahlen in Gew.-%)

| | 1-1 | 1-2 | 1-3 | 1-4 | 1-5 | 1-6 | 1-7 | 1-8 | 1-9 | 1-10 |
|---|---|---|---|---|---|---|---|---|---|---|
| Titanium dioxide | | 2 | 5 | | | | | | | 3 |
| [(7,8-Dihydroxy-4-oxo-4H-chromen-3-yl)-hydroxymethyl]-phosphonsäure | 5 | 3 | 2 | 1 | 2 | | | | 1 | 1 |
| [(6-Hydroxy-4-oxo-4H-chromen-3-yl)-hydroxymethyl]-phosphonsäure | | | | | | 1 | 2 | 1 | | |
| Zinc oxide | | | | | | | | 5 | 2 | |
| UV-Pearl , OMC | 30 | 15 | 15 | 15 | 15 | 15 | 15 | 15 | 15 | 15 |
| Polyglyceryl-3-Dimerate | 3 | 3 | 3 | 3 | 3 | 3 | 3 | 3 | 3 | 3 |
| Cera Alba | 0,3 | 0,3 | 0,3 | 0,3 | 0,3 | 0,3 | 0,3 | 0,3 | 0,3 | 0,3 |
| Hydrogenated Castor Oil | 0,2 | 0,2 | 0,2 | 0,2 | 0,2 | 0,2 | 0,2 | 0,2 | 0,2 | 0,2 |
| Paraffinium Liquidum | 7 | 7 | 7 | 7 | 7 | 7 | 7 | 7 | 7 | 7 |
| Caprylic/Capric Triglyceride | 7 | 7 | 7 | 7 | 7 | 7 | 7 | 7 | 7 | 7 |
| Hexyl Laurate | 4 | 4 | 4 | 4 | 4 | 4 | 4 | 4 | 4 | 4 |
| PVP/Eicosene Copolymer | 2 | 2 | 2 | 2 | 2 | 2 | 2 | 2 | 2 | 2 |
| Propylene Glycol | 4 | 4 | 4 | 4 | 4 | 4 | 4 | 4 | 4 | 4 |
| Magnesium Sulfate | 0,6 | 0,6 | 0,6 | 0,6 | 0,6 | 0,6 | 0,6 | 0,6 | 0,6 | 0,6 |
| Tocopherol | 0,5 | 0,5 | 0,5 | 0,5 | 0,5 | 0,5 | 0,5 | 0,5 | 0,5 | 0,5 |
| Tocopheryl Acetate | 0,5 | 0,5 | 0,5 | 0,5 | 0,5 | 0,5 | 0,5 | 0,5 | 0,5 | 0,5 |
| Cyclomethicone | 0,5 | 0,5 | 0,5 | 0,5 | 0,5 | 0,5 | 0,5 | 0,5 | 0,5 | 0,5 |
| Propylparabene | 0,05 | 0,05 | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 | 0,15 | 0,15 |
| Water | ad 100 | ad 100 | ad 100 | ad 100 | ad 100 | ad 100 | ad 100 | ad 100 | ad 100 | ad 100 |

| | 1-11 | 1-12 | 1-13 | 1-14 | 1-15 | 1-16 | 1-17 | 1-18 |
|---|---|---|---|---|---|---|---|---|
| Titanium dioxide | 3 | | 2 | | 3 | | 2 | 5 |

(fortgesetzt)

| | 1-11 | 1-12 | 1-13 | 1-14 | 1-15 | 1-16 | 1-17 | 1-18 |
|---|---|---|---|---|---|---|---|---|
| Benzylidene malonate polysiloxane | | 1 | 0,5 | | | | | |
| Methylene Bis-Benztriazolyl Tetramethylbutylphenol | 1 | 1 | 0,5 | | | | | |
| [(7,8-Dihydroxy-4-oxo-4H-chromen-3-yl)-hydroxymethyl]-phosphonsäure | 5 | 3 | 2 | 5 | 1 | 3 | 7 | 2 |
| Polyglyceryl-3-Dimerate | 3 | 3 | 3 | 3 | | | | |
| Cera Alba | 0,3 | 0,3 | 0,3 | 0,3 | 2 | 2 | 2 | 2 |
| Hydrogenated Castor Oil | 0,2 | 0,2 | 0,2 | 0,2 | | | | |
| Paraffinium Liquidum | 7 | 7 | 7 | 7 | | | | |
| Caprylic/Capric Triglyceride | 7 | 7 | 7 | 7 | | | | |
| Hexyl Laurate | 4 | 4 | 4 | 4 | | | | |
| PVP/Eicosene Copolymer | 2 | 2 | 2 | 2 | | | | |
| Propylene Glycol | 4 | 4 | 4 | 4 | | | | |
| Magnesium Sulfate | 0,6 | 0,6 | 0,6 | 0,6 | | | | |
| Tocopherol | 0,5 | 0,5 | 0,5 | 0,5 | | | | |
| Tocopheryl Acetate | 0,5 | 0,5 | 0,5 | 0,5 | 1 | 1 | 1 | 1 |
| Cyclomethicone | 0,5 | 0,5 | 0,5 | 0,5 | | | | |
| Propylparabene | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 |
| Dicocoyl Pentyerythrityl Citrate (and) Sorbitan Sesquioleate (and) Cera Alba (and) Aluminium Stearate | | | | | 6 | 6 | 6 | 6 |
| PEG-7 Hydrogenated Castor Oil | | | | | 1 | 1 | 1 | 1 |
| Zinc Stearate | | | | | 2 | 2 | 2 | 2 |
| Oleyl Erucate | | | | | 6 | 6 | 6 | 6 |
| Decyl Oleate | | | | | 6 | 6 | 6 | 6 |
| Dimethicone | | | | | 5 | 5 | 5 | 5 |
| Tromethamine | | | | | 1 | 1 | 1 | 1 |

(fortgesetzt)

| | 1-11 | 1-12 | 1-13 | 1-14 | 1-15 | 1-16 | 1-17 | 1-18 |
|---|---|---|---|---|---|---|---|---|
| Glycerin | | | | | 5 | 5 | 5 | 5 |
| Allantoin | | | | | 0,2 | 0,2 | 0,2 | 0,2 |
| Water | ad 100 | ad 100 | ad 100 | ad 100 | ad 100 | ad 100 | ad 100 | ad 100 |

Tabelle 2: O/W-Emulsionen, Zahlen in Gew.-%

| | 2-1 | 2-2 | 2-3 | 2-4 | 2-5 | 2-6 | 2-7 | 2-8 | 2-9 | 2-10 |
|---|---|---|---|---|---|---|---|---|---|---|
| Titanium dioxide | | 2 | 5 | | | | | | | 3 |
| Methylene Bis-Benztriazolyl Tetramethylbutylphenol | | | | | | 1 | 2 | 1 | | |
| [(7,8-Dihydroxy-4-oxo-4H-chromen-3-yl)-hydroxymethyl]-phosphonsäuredimethylester | 1 | 3 | | 3 | 2 | 5 | | 5 | 3 | 1 |
| [(7,8-Dihydroxy-4-oxo-4H-chromen-3-yl)-hydroxymethyl]-phosphonsäure | 5 | 5 | 5 | 5 | 5 | 5 | 5 | 5 | 5 | 5 |
| [(6-Hydroxy-4-oxo-4H-chromen-3-yl)-hydroxymethyl]-phosphonsäure | 1 | 5 | 4 | | 6 | | 7 | | 2 | 1 |
| 4-Methylbenzyliden Camphor | 2 | | 3 | | 4 | | 3 | | 2 | |
| BMDBM | 1 | 3 | | 3 | 3 | | 3 | 3 | 3 | |
| Stearyl Alcohol (and) Steareth-7 (and) Steareth-10 | 3 | 3 | 3 | 3 | 3 | 3 | 3 | 3 | 3 | 3 |
| Glyceryl Stearate (and) Ceteth-20 | 3 | 3 | 3 | 3 | 3 | 3 | 3 | 3 | 3 | 3 |
| Glyceryl Stearate | 3 | 3 | 3 | 3 | 3 | 3 | 3 | 3 | 3 | 3 |
| Microwax | 1 | 1 | 1 | 1 | 1 | 1 | 1 | 1 | 1 | 1 |
| Cetearyl Octanoate | 11,5 | 11,5 | 11,5 | 11,5 | 11,5 | 11,5 | 11,5 | 11,5 | 11,5 | 11,5 |
| Caprylic/Capric Triglyceride | 6 | 6 | 6 | 6 | 6 | 6 | 6 | 6 | 6 | 6 |
| Oleyl Oleate | 6 | 6 | 6 | 6 | 6 | 6 | 6 | 6 | 6 | 6 |
| Propylene Glycol | 4 | 4 | 4 | 4 | 4 | 4 | 4 | 4 | 4 | 4 |
| Glyceryl Stearate SE | | | | | | | | | | |
| Stearic Acid | | | | | | | | | | |
| Persea Gratissima | | | | | | | | | | |
| Propylparabene | 0,05 | 0,05 | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 | 0,15 | 0,15 |
| Tromethamine | | | 1,8 | | | | | | | |

(fortgesetzt)

| | 2-1 | 2-2 | 2-3 | 2-4 | 2-5 | 2-6 | 2-7 | 2-8 | 2-9 | 2-10 |
|---|---|---|---|---|---|---|---|---|---|---|
| Glycerin | | | | | | | | | | |
| Water | ad 100 | ad 100 | ad 100 | ad 100 | ad 100 | ad 100 | ad 100 | ad 100 | ad 100 | ad 100 |
| | | | 2-11 | 2-12 | 2-13 | 2-14 | 2-15 | 2-16 | 2-17 | 2-18 |
| Titanium dioxide | | | 3 | | 2 | | | | 2 | 5 |
| Benzylidene malonate polysiloxane | | | | 1 | 0,5 | | | | | |
| Methylene Bis-Benztriazolyl Tetramethylbutylphenol | | | 1 | 1 | 0,5 | | | | | |
| [(7,8-Dihydroxy-4-oxo-4H-chromen-3-yl)-hydroxymethyl]-phosphonsäuredimethylester | | | 5 | 5 | 5 | 5 | 5 | 5 | 5 | 5 |
| Zinc oxide | | | | | 2 | | | | | |
| UV-Pearl , OMC | | | 15 | 15 | 15 | 30 | 30 | 30 | 15 | 15 |
| 4-Methylbenzyliden Camphor | | | | | | 3 | | | | |
| BMDBM | | | | | | 1 | | | | |
| Phenylbenzimidazole Sulfonic Acid | | | | | | | 4 | | | |
| Stearyl Alcohol (and) Steareth-7 (and) Steareth-1 0 | | | 3 | 3 | 3 | 3 | | | | |
| Glyceryl Stearate (and) Ceteth-20 | | | 3 | 3 | 3 | 3 | | | | |
| Glyceryl Stearate | | | 3 | 3 | 3 | 3 | | | | |
| Microwax | | | 1 | 1 | 1 | 1 | | | | |
| Cetearyl Octanoate | | | 11,5 | 11,5 | 11,5 | 11,5 | | | | |
| Caprylic/Capric Triglyceride | | | 6 | 6 | 6 | 6 | 14 | 14 | 14 | 14 |
| Oleyl Oleate | | | 6 | 6 | 6 | 6 | | | | |
| Propylene Glycol | | | 4 | 4 | 4 | 4 | | | | |
| Glyceryl Stearate SE | | | | | | | 6 | 6 | 6 | 6 |
| Stearic Acid | | | | | | | 2 | 2 | 2 | 2 |
| Persea Gratissima | | | | | | | 8 | 8 | 8 | 8 |
| Propylparabene | | | 0,05 | 0,05 | 0,05 | 0,05 | 0,05 | 0,05 | 0,05 | 0,05 |

(fortgesetzt)

| | 2-11 | 2-12 | 2-13 | 2-14 | 2-15 | 2-16 | 2-17 | 2-18 |
|---|---|---|---|---|---|---|---|---|
| Methylparabene | 0,15 | 0,15 | 0,15 | 0,15 | 0,15 | 0,15 | 0,15 | 0,15 |
| Tromethamine | | | | | 1,8 | | | |
| Glycerin | | | | | 3 | 3 | 3 | 3 |
| Water | ad 100 | ad 100 | ad 100 | ad 100 | ad 100 | ad 100 | ad 100 | ad 100 |

Tabelle 3: Gele, Zahlen in Gew.-%

| | 3-1 | 3-2 | 3-3 | 3-4 | 3-5 | 3-6 | 3-7 | 3-8 | 3-9 | 3-10 |
|---|---|---|---|---|---|---|---|---|---|---|
| a = aqueaous gel | | | | | | | | | | |
| Titanium dioxide | | 2 | 5 | | | | | | | 3 |
| [(6-Hydroxy-4-oxo-4H-chromen-3-yl)-hydroxymethyl]-phosphonsäure | | | | 1 | 2 | | | | 1 | 1 |
| [(6-Hydroxy-4-oxo-4H-chromen-3-yl)-hydroxymethyl]-phosphonsäure, Kaliumsalz | 1 | 3 | | 2 | | 5 | | 5 | 2 | |
| [(7,8-Dihydroxy-4-oxo-4H-chromen-3-yl)-hydroxymethyl]-phosphonsäure | 5 | 5 | 5 | 5 | 5 | 5 | 5 | 5 | 5 | 5 |
| [(7,8-Dihydroxy-4-oxo-4H-chromen-3-yl)-hydroxymethyl]-phosphonsäure, Kaliumsalz | 1 | 5 | 4 | | 6 | | 7 | | 2 | 1 |
| Benzylidene malonate polysiloxane | | | 1 | 1 | 2 | | | | 1 | 1 |
| Methylene Bis-Benztriazolyl Tetramethylbutylphenol | | 1 | | | | 1 | 2 | 1 | | |
| Zinc oxide | | | | 2 | | | | 5 | 2 | |
| UV-Pearl Ethylhexyl Mehtoxycinnamat | 30 | 15 | 15 | 15 | 15 | 15 | 15 | 15 | 15 | 15 |
| 4-Methylbenzyliden Camphor | | | | | 2 | | | | | |
| Butylmethoxydibenzoylmethane | | 1 | | | | | | | | |
| Phenylbenzimidazole Sulfonic Acid | | | 4 | | | | | | | |
| Prunus Dulcis | 5 | 5 | 5 | 5 | 5 | 5 | 5 | 5 | 5 | 5 |
| Tocopheryl Acetate | 0,5 | 0,5 | 0,5 | 0,5 | 0,5 | 0,5 | 0,5 | 0,5 | 0,5 | 0,5 |
| Caprylic/Capric Triglyceride | 3 | 3 | 3 | 3 | 3 | 3 | 3 | 3 | 3 | 3 |
| Octyldodecanol | 2 | 2 | 2 | 2 | 2 | 2 | 2 | 2 | 2 | 2 |
| Decyl Oleate | 2 | 2 | 2 | 2 | 2 | 2 | 2 | 2 | 2 | 2 |
| PEG-8 (and) Tocopherol (and) Ascorbyl Palmitate (and) Ascorbic Acid (and) Citric Acid | 0,05 | 0,05 | 0,05 | 0,05 | 0,05 | 0,05 | 0,05 | 0,05 | 0,05 | 0,05 |
| Sorbitol | 4 | 4 | 4 | 4 | 4 | 4 | 4 | 4 | 4 | 4 |
| Polyacrylamide (and) C13-14 Isoparaffin (and) Laureth-7 | 3 | 3 | 3 | 3 | 3 | 3 | 3 | 3 | 3 | 3 |

(fortgesetzt)

| | 3-1 | 3-2 | 3-3 | 3-4 | 3-5 | 3-6 | 3-7 | 3-8 | 3-9 | 3-10 |
|---|---|---|---|---|---|---|---|---|---|---|
| Propylparabene | 0,05 | 0,05 | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 | 0,15 | 0,15 |
| Tromethamine | | | 1,8 | | | | | | | |
| Water | ad 100 | ad 100 | ad 100 | ad 100 | ad 100 | ad 100 | ad 100 | ad 100 | ad 100 | ad 100 |

## Patentansprüche

**1.** Verbindungen der Formel I

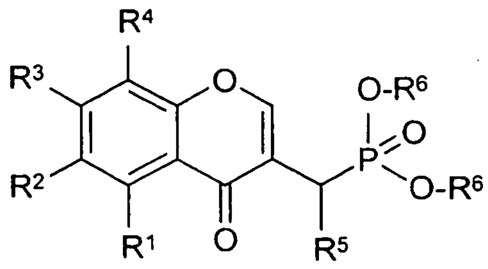

I

,

wobei
$R^1$ bis $R^3$ jeweils unabhängig voneinander H, Hydroxy oder Alkoxy mit 1 bis 8 C-Atomen bedeuten,
$R^4$ Alkyl mit 1 bis 4 C-Atomen, H, Hydroxy oder Alkoxy mit 1 bis 8 C-Atomen bedeutet,
$R^5$ H oder Hydroxy bedeutet und
$R^6$ H oder Alkyl mit 1 bis 18 C-Atomen bedeutet
oder deren Salze, wobei jedoch nicht alle $R^1$ bis $R^4$ zusammen gleich H sein dürfen.

**2.** Verbindungen nach Anspruch 1, **dadurch gekennzeichnet, dass** der Substituent $R^5$ Hydroxy bedeutet.

**3.** Verfahren zur Herstellung der Verbindungen der Formel I nach Anspruch 1 oder 2, wobei $R^6$ unabhängig voneinander Alkyl mit 1 bis 18 C-Atomen und $R^5$ = OH bedeutet, **dadurch gekennzeichnet, dass** man eine Verbindung der Formel II

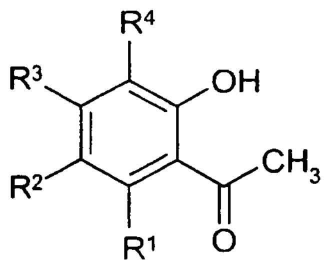

II

,

wobei $R^1$ bis $R^4$ eine der zuvor genannten Bedeutungen haben können, mit einem Reagenz ausgewählt aus $POCl_3$, Phosgen oder Trifluorsulfonsäureanhydrid und einem Arylalkyl-, Diaryl- oder Dialkylformamid zu einem Zwischenprodukt der Formel III

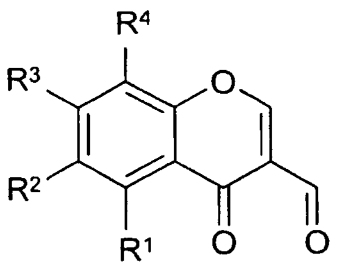

III

umsetzt, wobei $R^1$ bis $R^4$ eine der Bedeutungen nach Anspruch 1 haben,
und dieses anschließend mit
einem Phosphorigsäuredialkylester umsetzt.

4. Verfahren zur Herstellung von Verbindungen der Formel I, in denen $R^6$ H bedeutet, **dadurch gekennzeichnet, dass** nachfolgend zu dem Verfahren nach Anspruch 3 eine Esterhydrolyse durchgeführt wird und gegebenenfalls die erhaltenen Verbindungen in ihre Salze überführt werden.

5. Verfahren zur Herstellung von Verbindungen der Formel I, in denen $R^5$ H bedeutet, **dadurch gekennzeichnet, dass** nachfolgend zu dem Verfahren nach Anspruch 4 eine Reduktion durchgeführt wird.

6. Zubereitung, enthaltend zumindest eine Verbindung der Formel I nach Anspruch 1 oder 2.

7. Zubereitung nach Anspruch 6, **dadurch gekennzeichnet, dass** die Zubereitungen mindestens eine Verbindung der Formel I in einer Menge von 0,01 Gew.-% bis 20 Gew.-%, vorzugsweise in einer Menge von 0,1 Gew.-% bis 10 Gew.-% enthält.

8. Zubereitung nach Anspruch 6 oder 7, **dadurch gekennzeichnet, dass** sie ein oder mehrere weitere Antioxidantien und/oder Vitamine, enthält.

9. Zubereitung nach einem oder mehreren der Ansprüche 6 bis 8, wobei die Zubereitung einen oder mehrere UV-Filter enthält.

10. Zubereitung nach einem oder mehreren der Ansprüche 6 bis 8, **dadurch gekennzeichnet, dass** die Zubereitung ein Nahrungsmittel oder ein Nahrungsergänzungsmittel ist und einen für Nahrungsmittel geeigneten Träger sowie gegebenenfalls weitere Nahrungsergänzungstoffe enthält.

11. Zubereitung nach einem oder mehreren der Ansprüche 6 bis 9, **dadurch gekennzeichnet, dass** die Zubereitung einen für pharmazeutische, dermatologische oder kosmetische Anwendungen geeigneten Träger enthält.

12. Verfahren zur Herstellung einer Zubereitung nach einem oder mehreren der Ansprüche 6 bis 11, **dadurch gekennzeichnet, dass** zumindest eine Verbindung der Formel I mit einem pharmazeutisch, kosmetisch oder dermatologisch oder für Nahrungsmittel geeignetem Träger vermischt wird.

13. Verwendung der Verbindungen der Formel I nach Anspruch 1 oder 2 zur Herstellung einer Zubereitung mit antioxidanten Eigenschaften.

14. Verwendung der Verbindungen der Formel I nach Anspruch 1 oder 2 zur Herstellung von Zubereitungen, die eine schützende Wirkung gegen oxidativen Stress auf Körperzellen ausüben und/oder einer Alterung der Haut entgegenwirken bzw. zur Minderung der Folgen der Hautalterung beitragen können

## Claims

1. Compounds of the formula I

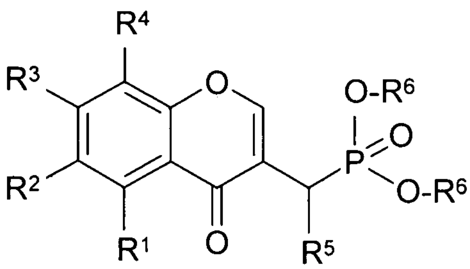

I

,

where
$R^1$ to $R^3$ each, independently of one another, denote H, hydroxyl or alkoxy having 1 to 8 C atoms,
$R^4$ denotes alkyl having 1 to 4 C atoms, H, hydroxyl or alkoxy having 1 to 8 C atoms,
$R^5$ denotes H or hydroxyl, and

$R^6$ denotes H or alkyl having 1 to 18 C atoms,
or salts thereof, but where all $R^1$ to $R^4$ together cannot be equal to H.

**2.** Compounds according to Claim 1, **characterised in that** the substituent $R^5$ denotes hydroxyl.

**3.** Process for the preparation of the compounds of the formula I according to Claim 1 or 2, where $R^6$, independently of one another, denotes alkyl having 1 to 18 C atoms and $R^5$ = OH, **characterised in that** a compound of the formula II

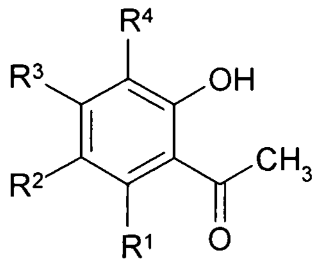

II

,

where $R^1$ to $R^4$ can have one of the meanings mentioned above, is reacted with a reagent selected from $POCl_3$, phosgene or trifluorosulfonic anhydride and an arylalkyl-, diaryl- or dialkylformamide to give an intermediate of the formula III

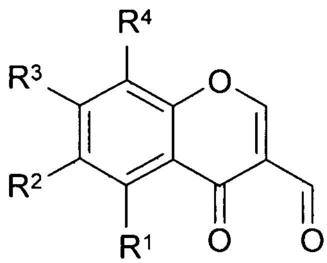

III

where $R^1$ to $R^4$ have one of the meanings according to Claim 1,
and this is subsequently reacted with a phosphonous acid dialkyl ester.

**4.** Process for the preparation of compounds of the formula I in which $R^6$ denotes H, **characterised in that** an ester hydrolysis is carried out after the process according to Claim 3, and the compounds obtained are optionally converted into their salts.

**5.** Process for the preparation of compounds of the formula I in which $R^5$ denotes H, **characterised in that** a reduction is carried out after the process according to Claim 4.

**6.** Composition comprising at least one compound of the formula I according to Claim 1 or 2.

**7.** Composition according to Claim 6, **characterised in that** the composition comprises at least one compound of the formula I in an amount of 0.01% by weight to 20% by weight, preferably in an amount of 0.1% by weight to 10% by weight.

**8.** Composition according to Claim 6 or 7, **characterised in that** it comprises one or more further antioxidants and/or vitamins.

**9.** Composition according to one or more of Claims 6 to 8, where the composition comprises one or more UV filters.

**10.** Composition according to one or more of Claims 6 to 8, **characterised in that** the composition is a food or food supplement and comprises a vehicle which is suitable for foods and optionally further food supplements.

**11.** Composition according to one or more of Claims 6 to 9, **characterised in that** the composition comprises a vehicle

which is suitable for pharmaceutical, dermatological or cosmetic applications.

**12.** Process for the preparation of a composition according to one or more of Claims 6 to 11, **characterised in that** at least one compound of the formula I is mixed with a vehicle which is suitable pharmaceutically, cosmetically or dermatologically or for foods.

**13.** Use of the compounds of the formula I according to Claim 1 or 2 for the preparation of a composition having antioxidant properties.

**14.** Use of the compounds of the formula I according to Claim 1 or 2 for the preparation of compositions which can exert a protective action against oxidative stress on body cells and/or can counter ageing of the skin or can contribute to a reduction in the consequences of skin ageing.

## Revendications

**1.** Composés de formule I

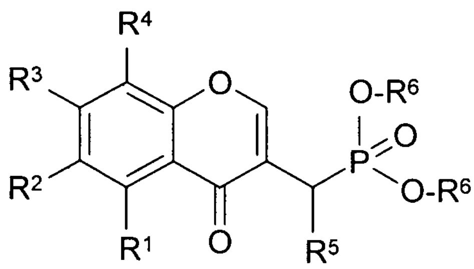

I

,

dans laquelle
$R^1$ à $R^3$ désignent chacun, indépendamment les uns des autres, H, hydroxyle ou alcoxy ayant de 1 à 8 atomes de C,
$R^4$ désigne alkyle ayant de 1 à 4 atomes de C, H, hydroxyle ou alcoxy ayant de 1 à 8 atomes de C,
$R^5$ désigne H ou hydroxyle, et
$R^6$ désigne H ou alkyle ayant de 1 à 18 atomes de C,
ou des sels de ceux-ci, mais où tous les $R^1$ à $R^4$ ne peuvent pas simultanément être H.

**2.** Composés selon la revendication 1, **caractérisés en ce que** le substituant $R^5$ désigne hydroxyle.

**3.** Procédé de préparation des composés de formule I selon la revendication 1 ou 2, où $R^6$, indépendamment les uns des autres, désigne alkyle ayant de 1 à 18 atomes de C et $R^5$ = OH, **caractérisé en ce qu'**un composé de formule II

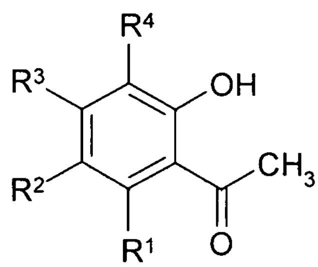

II

,

où $R^1$ à $R^4$ peuvent revêtir l'une des significations mentionnées ci-dessus, est réagi avec un réactif sélectionné parmi $POCl_3$, le phosgène ou l'anhydride trifluorosulfonique et un arylalkyl-, diaryl- ou dialkylformamide pour donner un intermédiaire de formule III

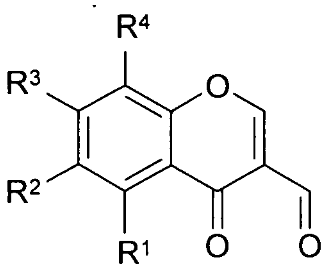

III

,

où $R^1$ à $R^4$ ont l'une des significations selon la revendication 1,
et ceci est réagi ensuite avec un ester dialkylique d'acide phosphoneux.

**4.** Procédé de préparation de composés de formule I dans laquelle $R^6$ désigne H, **caractérisé en ce qu'**une hydrolyse d'ester est réalisée après le procédé selon la revendication 3, et les composés obtenus sont éventuellement convertis en leurs sels.

**5.** Procédé de préparation de composés de formule I dans laquelle $R^5$ désigne H, **caractérisé en ce qu'**une réduction est réalisée après le procédé selon la revendication 4.

**6.** Composition comprenant au moins un composé de formule I selon la revendication 1 ou 2.

**7.** Composition selon la revendication 6, **caractérisée en ce que** la composition comprend au moins un composé de formule I en une quantité de 0,01% en poids à 20% en poids, de préférence en une quantité de 0,1% en poids à 10% en poids.

**8.** Composition selon la revendication 6 ou 7, **caractérisée en ce qu'**elle comprend un ou plusieurs antioxydants et/ou vitamines supplémentaires.

**9.** Composition selon l'une ou plusieurs des revendications 6 à 8, où la composition comprend un ou plusieurs filtres UV.

**10.** Composition selon l'une ou plusieurs des revendications 6 à 8, **caractérisée en ce que** la composition est un aliment ou un complément alimentaire et comprend un véhicule qui est convenable pour les aliments et éventuellement d'autres compléments alimentaires.

**11.** Composition selon l'une ou plusieurs des revendications 6 à 9, **caractérisée en ce que** la composition comprend un véhicule qui est convenable pour des applications pharmaceutiques, dermatologiques ou cosmétiques.

**12.** Procédé de préparation d'une composition selon l'une ou plusieurs des revendications 6 à 11, **caractérisé en ce qu'**au moins un composé de formule I est mélangé avec un véhicule qui est pharmaceutiquement, cosmétiquement ou dermatologiquement convenable, ou convenable pour les aliments.

**13.** Utilisation des composés de formule I selon la revendication 1 ou 2 pour la préparation d'une composition ayant des propriétés antioxydantes.

**14.** Utilisation des composés de formule 1 selon la revendication 1 ou 2 pour la préparation de compositions pouvant exercer une action protectrice contre le stress oxydatif sur les cellules de l'organisme et/ou pouvant combattre le vieillissement cutané ou pouvant contribuer à une réduction des conséquences du vieillissement cutané.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- JP 10237081 A
- US 6242099 B1
- WO 0009652 A
- WO 0072806 A
- WO 0071084 A
- EP 0671161 A
- DE 4116123 A
- DE OS4308282 A
- WO 20040092283 A
- WO 2004091567 A
- EP 1044676 A
- EP 0928608 A


### In der Beschreibung aufgeführte Nicht-Patentliteratur


- Antioxidantien zu den Zubereitungen. Laut CD Römpp Chemie Lexikon. Georg Thieme Verlag, 1995
- **Kostka et al.** *Phosphorus, Sulfur and Silicon and the related elements,* 1991, vol. 57 (3-4), 279-85
- **C. Sanchez-Moreno ; J.A. Larrauri ; F. Saura-Calixto.** *J. Sci. Food Agric.,* 1998, vol. 76 (2), 270-276
- **E. A. Galinski et al.** *Eur. J. Biochem.,* 1985, vol. 149, 135-139
- **Buenger, J. ; Ackermann, H. ; Jentzsch, A. ; Mehling, A. ; Pfizner, I. ; Reiffen, K.-A. ; Schroeder, K.-R. ; Wollenweber U.** An interlaboratory comparison of methods used to assess antioxidant potentials. *Int. J. Cosm. Sci.,* 2006, vol. 28, 1-12